# EUROPEAN PATENT SPECIFICATION

(11) **EP 0 962 463 B1**
(45) Date of publication and mention of the grant of the patent: **10.07.2002**
(21) Application number: 99109726.2
(22) Date of filing: 20.12.1990
(51) Int. Cl.: C07H 21/00, C12Q 1/68, C07K 14/00

(54) **Uncharged morpholino-based polymers having phosphorus-containing chiral intersubunit linkages**
Ungeladene, auf Morpholin basierende Polymere mit Chiralen, Phosphor enthaltenden Brücken zwischen den Untereinheiten
Polymeres non chargés à base de Morpholine ayant des liaisons chirales phosphoreuses entre les sous-unités

(30) Priority: 20.12.1989 US 454057
(43) Date of publication of application: 08.12.1999
(62) Divisional of application: 91902085.9
(73) Proprietor: ANTIVIRALS INC., Portland, OR 97258 (US)
(72) Inventor: Summerton, James E., Corvallis, OR 97330 (US); Weller, Dwight D., Corvallis, OR 97330 (US)
(74) Representative: Goldin, Douglas Michael

(56) References cited:
- WO-A-86/05518
- WO-A-86/05519
- STIRCHAK E P ET AL: "UNCHARGED STEREOREGULAR NUCLEIC ACID ANALOGS: 2. MORPHOLINO NUCLEOSIDE OLIGOMERS WITH CARBAMATE INTERNUCLEOSIDE LINKAGES" NUCLEIC ACIDS RESEARCH, vol. 17, no. 15, 1 January 1989 (1989-01-01), pages 6129-6141, XP002070310 ISSN: 0305-1048

## Description

### Field of the Invention

The present invention relates to morpholino-based polymers.

### References

Agarwal, Proc Nat Acad Sci USA, 85:7079 (1988).
Atencio, E.J., et al. (Editors), Nucleotide Sequences: a compilation from GENBANK and EMBL data libraries, Vol. 6 Viruses, Academic Press (1986-87).
Balgobin, N., et al., Tetrahedron Lett, 22:3667 (1981).
Belikova, Tetrahedron Lett, 37:3557 (1967).
Blake et al., Biochem, 24:6132 (1985a).
Blake et al., Biochem 24:6139 (1985b).
Bower et al., Nucleic Acids Res. 15:4915 (1987).
Dikshit et al., Canadian J Chem, 66:2989 (1988).
Froehler, et al., Nucleic Acids Res. 16:4831 (1988).
Fox, J.J., et al., J Am Chem Soc, 80:1669 (1958).
Gait, "Oligonucleotide Synthesis A Practical "Approach," pp. 31-33, IRL Press (Oxford, England) (1984).
Griffen, et al., Biomed. & Environ. Mass Spectrometry 17:105 (1967).
Goldberg, M. L. et al; Methods in Enzymology 68:206 (1979).
Greenlee, J Org Chem, 49 2632 (1984).
Grunstein, M. et al; Methods in Enzymology 68:379 (1979).
Himmelsbach, F., and W. Pfleiderer, Tetrahedron Lett, 24:3583 (1983).
Jayaraman, et al., Proc Natl Acad Sci USA 78:1537 (1981).
Kamimura et al., Chem Lett (The Chem. Soc. of Japan) pg. 1051 (1983)
LaPlanche et al., Nucleic Acids Res, 14: 9081 (1986).
Lerman, L.S., "DNA Probes: Applications in Genetic and Infectious Disease and Cancer,"Current Comm in Molec Biol (Cold Spring Harbor Laboratory) (1986).
Letsinger and Miller, J Amer Chem Soc, 91:3356 (1969).
Myers, G., et al. (Editors), Human Retroviruses and AIDS: A compilation and analysis of amino acid and nucleic acid sequences, Theoretical Biology and Biophysics Group, Los Alamos NM (LAUR90-1393) (1990).
McBride et al., J Amer Chem Soc 108:2040 (1986).
Miller, et al., Biochemistry 18:5134 (1979).
Miller, et al., J Biol Chem 255:6959 (1980).
Miller, et al., Biochimie 67:769 (1985).
Murakami, et al., Biochemistry 24:4041 (1985).
Niedballa, U., and H. Vorbruggen, J Org Chem, 39:3668 (1974).
Pitha, Biochem Biophys Acta 204:39 (1970a).
Pitha, Biopolymers 9:965 (1970b).
Reese, C.B., and R.S. Saffhill, J Chem Soc PerkinTrans, 1:2937 (1972).
Smith, et al., J.A.C.S. 80:6204 (1958).
Smith, et al., Proc Natl Acad Sci USA 83:2787 (1986). Southern, E.; Methods in Enzymology 68:152 (1979)
Stirchak E.P. et al., Organic Chem. 52:4202 (1987).
Summerton, J., et al., J Molec Biol 122:145 (1978)
Summerton, J., et al., J Molec Biol 78:61 (1979a).
Summerton, J., J Molec Biol 78:77 (1979b).
Szostak, J. W. et al; Methods in Enzymology 68:419 (1979).
Thomas, P.; Methods in Enzymology 100:255 (1983).
Toulme et al., Proc Nat Acad Sci USA, 83:1227 (1986).
Trichtinger et al., Tetrahedron Lett 24:711 (1983).

### Background of the Invention

Polymers which are designed for base-specific binding to polynucleotides have significant potential both for in vitro detection of specific genetic sequences characteristic of pathogens and for in vivo inactivation of genetic sequences causing many diseases -- particularly viral diseases.

Standard ribo- and deoxyribonucleotide polymers have been widely used both for detection of complementary genetic sequences, and more recently, for inactivating targeted genetic sequences. However, standard polynucleotides suffer from a number of limitations when used for base-specific binding to target oligonucleotides. These limitations include (i) restricted passage across biological membranes, (ii) nuclease sensitivity, (ii) target binding which is sensitive to ionic concentration, and (iv) susceptibility to cellular strand-separating mechanisms.

In principle, the above limitations can be overcome or minimized by designing polynucleic acid analogs in which the bases are linked along an uncharged backbone. Examples of uncharged nucleic acid analogs have been reported. Pitha et al (1970a, b) have disclosed a variety of homopolymeric polynucleotide analogs in which the normal sugar-phosphate backbone of nucleic acids is replaced by a polyvinyl backbone. These nucleic acid analogs were reported to have the expected Watson/Crick pairing specificities with complementary polynucleotides, but with substantially reduced Tm values (Pitha, 1970a). One serious limitation of this approach is the inability to construct polymers by sequential subunit addition, for producing polymers with a desired base sequence. Thus the polymers cannot be used for base-specific binding to selected target sequences. Polynucleotide analogs containing uncharged, but stereoisomeric, methylphosphonate linkages between the deoxyribonucleoside subunits have also been reported (Miller, 1979, 1980; Jayaraman; Murakami; Blake, 1985a, 1985b; Smith). More recently, a variety of analogous uncharged phosphoramidate-linked oligonucleotide analogs have also been reported (Froehler, 1988). These polymers comprise deoxynucleosides linked by the 3'OH group of one subunit and the 5'OH group of another subunit via an uncharged chiral phosphorus-containing group. These compounds have been shown to bind to and selectively block single-strand polynucleotide target sequences. However, uncharged phosphorus-linked polynucleotide analogs using deoxyribonucleoside subunits are particularly costly and difficult to prepare; the subunit starting material is quite costly and of limited availability.

More recently, deoxyribonucleotide analogs having uncharged and achiral subunit linkages have been constructed (Stirchak 1987). These uncharged, achiral deoxyribonucleoside-derived analogs are, as mentioned above, limited by relatively high cost of starting materials.

### Summary of the Invention

It is one general object of the invention to provide a polymer capable of sequence-specific binding to polynucleotides and which overcomes or minimizes many of the problems and limitations associated with polynucleotide analog polymers noted above.

The invention provides a polymer comprised of morpholino subunit structures of the form: 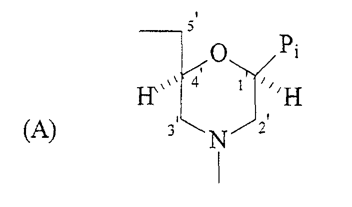 where (i) the structures are linked together by uncharged, chiral linkages, joining the morpholino nitrogen of one subunit to the 5' exocyclic carbon of an adjacent subunit, and (ii) Pᵢ is a purine or pyrimidine base-pairing moiety effective to bind by base-specific hydrogen bonding to a base in a polynucleotide, and wherein the linkage (i) is 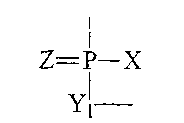 wherein X is F, CH₂R, OCH₂R, -S-CH₂R, NR₁R₂, morpholino, pyrrolyl or pyrazolyl; where R, R₁ and R₂ may be the same or different and are selected from H and CH₃; Y₁ is joined to the 5'-exocyclic carbon of the morpholino subunit; Y₁ is O, S, CH₂ or NR; and Z is O or S.

Typically, Z is O and Y₁ is O.

### Brief Description of the Figures

Figure 1 shows a basic β-morpholino ring structure which is linked through uncharged, phosphorus-containing chiral linkages to form the polymer of the present invention. Pᵢ is a purine or pyrimidine base pairing moiety.

Figure 2 shows several exemplary purine- and pyrimide base-pairing moieties (represented as Pᵢ of the ring structures shown in Figure 1), where X = H, CH₃, F, Cl, Br, or I. Typically, in the polymer of the present invention, Pᵢ is selected from the groups shown in Figure 2.

Figure 3 shows the subunit suitable for forming the polymers, where X, Y and Z are as defined above.

Figure 4 shows a repeating subunit segment of the polymers of the invention. X, Y, and Z are as in Figure 3. Pᵢ and Pⱼ are purine or pyrimidine base pairing moieties.

Figure 5 shows the steps in the synthesis of several types of the morpholino subunits from a ribonucleoside.

Figure 6 shows an alternative synthesis of the basic morpholino subunit.

Figure 7 shows the binding mode for 2-amine-containing purines to polar major-groove sites of respective target base-pairs (Figure 7a) and a representative base sequence of a duplex-binding polymer (Figure 7b). In Figure 7b, a = Adenine; c = cytosine; g = guanine; t = thymine; u = uracil; D = 2,6-Diaminopurine or 2-aminopurine; G = Guanine or thioguanine; | = high specificity hydrogen bonding; and : = low specificity hydrogen bonding.

Figure 8 shows two methods for linking morpholino subunits through a phosphoramidate linkage.

Figure 9 shows the thermal denaturation plot for a (morpholino) (C)₆/poly(G) complex, where the (morpholino) (C)₆ polymer was constructed according to the present invention.

Figure 10 illustrates the use of a morpholino polymer in a probe-diagnostic system.

### Detailed Description of the Invention

The polymer of the present invention is designed for base-specific binding to a target sequence of a polynucleotide.

### A. Morpholino-Based Subunits

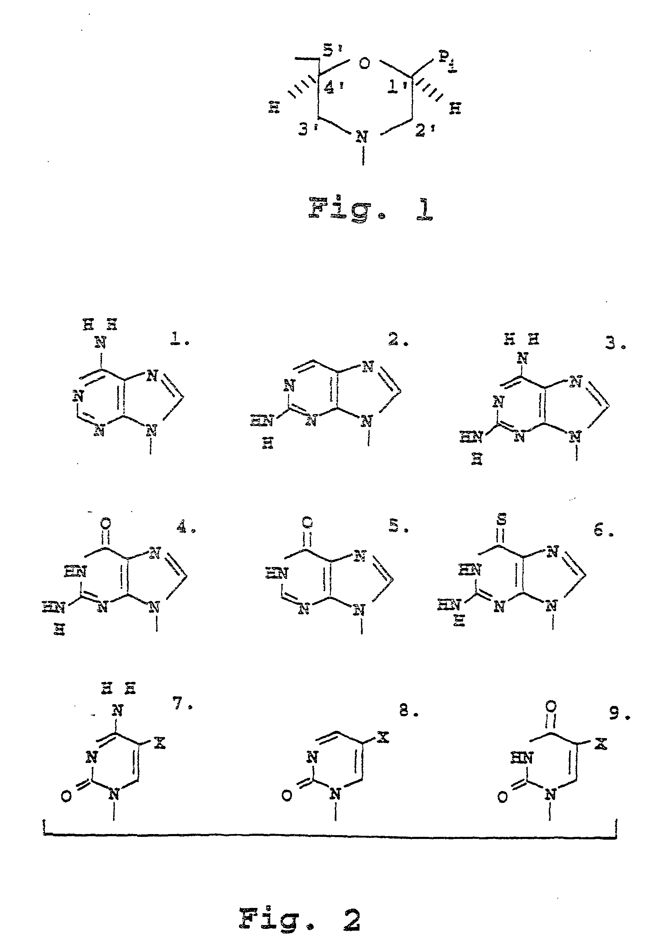

Figure 1 shows the β-morpholino ring structures on which the polymer subunits are based, where the morpholino carbon atoms are numbered as in the parent ribose. As seen in Figure 1, the ring structure contains a 5' methylene attached to the 4' carbon in the β-orientation. Typically, the polymer of the present invention is composed of at least 3 morpholino subunits.

Each ring structure includes a purine or pyrimidine moiety, Pᵢ, attached to the backbone morpholine moiety through a linkage in the β-orientation.

The purine hydrogen-bonding moieties or bases include purines as well as purine-like planar ring structures having a 5-6 fused ring in which one or more of the atoms, such as N3, N7, or N9 is replaced by a suitable atom, such as carbon, or the C8 is replaced by a nitrogen. The pyrimidine moieties likewise include pyrimidines as well as pyrimidine-like planar 6-membered rings in which one or more of the atoms, such as N1, is replaced by a suitable atom, such as carbon. Preferred hydrogen-bonding moieties in the invention include the set of purines and pyrimidines shown in Figure 2. Each base includes at least two hydrogen-bonding sites specific for a polynucleotide base or base-pair. Where the polymers are used for sequence-specific binding to single-stranded polynucleotides, the purine structures 1 through 3 are designed to bind to thymine or uracil bases; structures 7-8, to guanine bases; structures 4-6, to cytosine bases; and structures 9, to adenine bases.

Typically, at least one of the Pᵢ moieties is a 2,6-diaminopurine moiety. Typically, at least one of the Pᵢ moieties is a 5-halouracil moiety. Typically, at least 70% of the Pᵢ moieties are 2-amine containing purine moieties.

The polymers of the invention are also effective to bind to hydrogen-bonding sites accessible through the major-groove in duplex polynucleotides having mostly purine bases in one strand and mostly pyrimidine bases in the complementary strand, as discussed below.

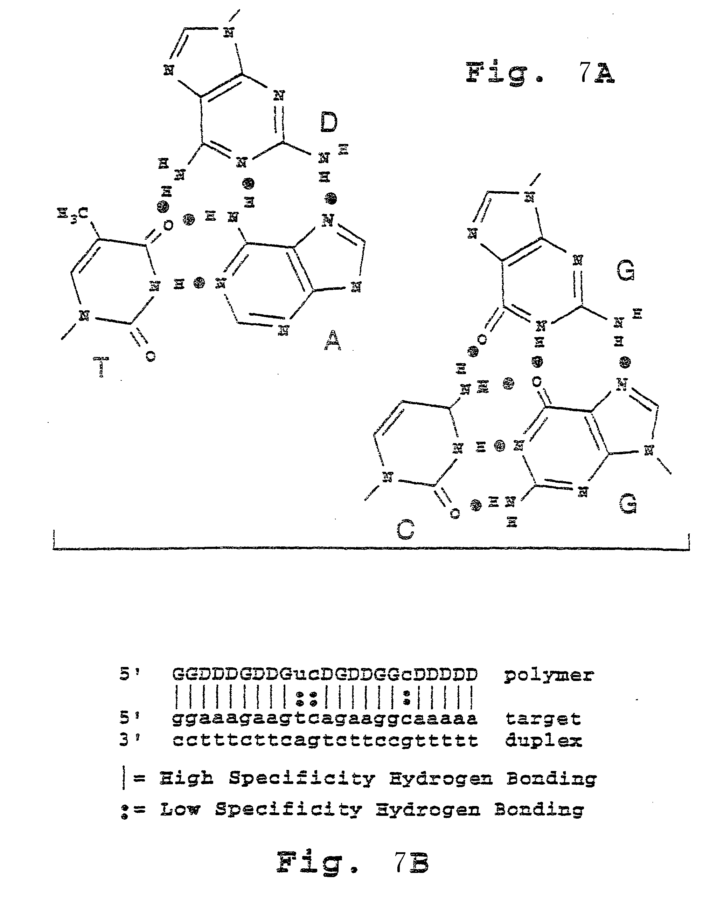

Because of the similar type and positioning of the two central polar major-groove sites among the different base-pairs of duplex nucleic acids (i.e., the NH4 and O6 of a CG base-pair present the same H-bonding array as the NH6 and O4 of an AT base-pair), the H-bonding moiety of a duplex-binding polymer must hydrogen-bond to the N7 of its target base-pair in order to uniquely recognize a given base-pair in a target genetic duplex. Thus, in the polymers of the present invention, which are targeted against duplex genetic sequences containing predominantly purines in one strand and predominantly pyrimidines in the other strand, the hydrogen-bonding moieties of the polymer preferably contain purines having an amine at the 2 position since that amine is suitably positioned for H-bonding to the N7 of the target base-pair. More specifically, Structures 2 and 3 of Figure 2 provide for specific binding to a TA or UA base-pair while Structures 4 and 6 provide for specific binding to a CG base-pair. Two bases which are particularly useful in a duplex-binding polymer are 2,6-diaminopurine (structure 3) and guanine (structure 4). Figure 7A illustrates the binding of these two bases to the polar major-groove sites of their respective target base-pairs in duplex nucleic acids. Figure 7B illustrates a representative base sequence of a polymer designed for binding a target genetic sequence in the duplex state.

The morpholino subunits of the instant invention are combined to form polymers by linking the subunits through stable, chiral, uncharged linkages of the formula 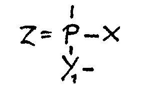 .

The selection of subunit linking groups for use in polymer synthesis is guided by several considerations. Initial screening of promising intersubunit linkages (i.e., those linkages which are predicted to not be unstable and which allow either free rotation about the linkage or which exist in a single conformation) typically involves the use of space-filling CPK or computer molecular models of duplex DNA or RNA. The DNA and RNA duplexes are constructed according to parameters determined by x-ray diffraction of oligodeoxyribonucleotides in the B-form and oligoribonucleotide-containing duplexes in the A-form.

In each of these constructed duplexes, one of the two sugar phosphate backbones is removed, and the prospective backbone, including the morpholino ring and intersubunit linkage, is replaced, if possible, on the sites of the bases from which the original sugar-phosphate backbone has been removed. Each resulting polynucleotide/polymer duplex is then examined for coplanarity of the Watson/Crick base pairs, torsional and angle strain in the prospective binding polymer backbone, degree of distortion imposed on the nucleic acid strand, and interstrand and intrastrand nonbonded interactions.

Initial studies of this type carried out in support of the invention show that a morpholino-based polymer has a preferred unit backbone length (i.e., the number of atoms in a repeating backbone chain in the polymer) of 6 atoms.

Since the morpholino structure itself contributes 4 atoms to each repeating backbone unit, the linkages in the six-atom repeating-unit backbone contributes two atoms to the backbone length. In all cases, the linkage between the ring structures is (a) uncharged, (b) chiral, (c) stable, and (d) must permit adoption of a conformation suitable for binding to the target polynucleotide.

Subunit backbone structures judged acceptable in the above modeling studies are then assessed for feasibility of synthesis. The actual chemical stability of the intersubunit linkage is often assessed with model compounds or dimers.

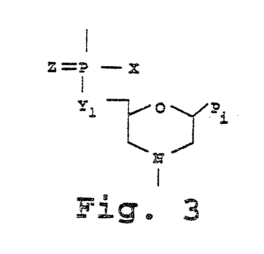

Figure 3 shows several the β-morpholino subunit type used in the present invention including linkage group, which meets the constraints and requirements outlined above.

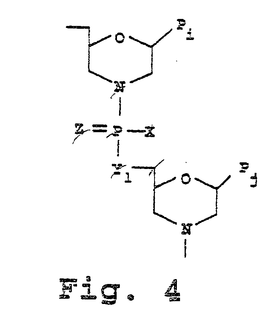

The subunit shown in Figure 3 is designed for 6-atom repeating-unit backbones, as shown in Figure 4. The Y₁ group linking the 5' morpholino carbon to the phosphorous group may be sulfur, NR wherein R is as defined above, CH₂ or, prefereably, oxygen. The X moiety pendant from the phosphorous is as defined above.

Typically, in the polymer of the invention, the linkage is of the form 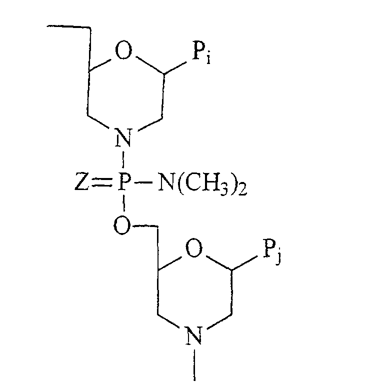 wherein Pⱼ is a purine or pyrimidine base-pairing moiety effective to bind by base specific hydrogen bonding to a base in a polynucleotide.

Another typical linkage is a linkage is of the form: 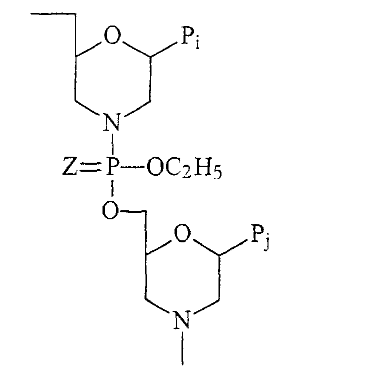 wherein Pⱼ is a purine or pyrimidine base-pairing moiety effective to bind by base specific hydrogen bonding to a base in a polynucleotide.

### B. Subunit Synthesis

The most economical starting materials for the synthesis of morpholino-subunits are generally ribonucleosides. Typically, ribonucleosides containing hydrogen-bonding moieties or bases (i.e., A, U, G, C) are synthesized to provide a complete set of subunits for polymer synthesis. Where a suitable ribonucleoside is not available, a 1-haloribose or, preferably, a 1α-bromoglucose derivative, can be linked to a suitable base and this nucleoside analog then converted to the desired β-morpholino structure via periodate cleavage, and closing the resultant dialdehyde on a suitable amine.

Because of the reactivity of the compounds used for subunit synthesis, activation, and/or coupling, it is generally desirable, and often necessary, to protect the exocyclic ring nitrogens of the bases. Selection of these protective groups is determined by (i) the relative reactivity of the nitrogen to be protected, (ii) the type of reactions involved in subunit synthesis and coupling, and (iii) the stability of the completed polymer prior to base deprotection.

Methods for base protecting a number of the more common ribonucleosides are given in Example 1. The methods detailed in the example are generally applicable for forming nucleosides with amine-protective groups. Standard base- protective groups used for nucleic acid chemistry are often suitable including the following groups: benzoyl for the N4 of C; benzoyl or p-nitrobenzoyl for the N6 of adenine (A); acetyl, phenylacetyl or isobutyryl for the N2 of guanine (G); and N2,N6-bis(isobutyryl) for 2,6-diaminopurine residues. These protective groups can be removed after polymer assembly by treatment with ammonium hydroxide.

It is sometimes desirable to protect the base portion of the morpholino subunit with a group which can be readily removed by other than a nucleophilic base. Suitable base protective groups removable by a strong non-nucleophilic base via a β-elimination mechanism include: 2-(4-nitrophenyl)ethoxy carbonyl or 2-(phenyl sulfonyl)ethoxycarbonyl for both the N4 of C and the N6 of A; and the 9-fluorenyl methoxycarbonyl for the N2 of G and the N2 and N6 of 2,6-diaminopurine. These groups can be removed after polymer assembly by treatment with the strong nonnucleophilic base 1,8-diazabicyclo[5.4.0]-undec-7-ene (DBU), under stringently anhydrous conditions.

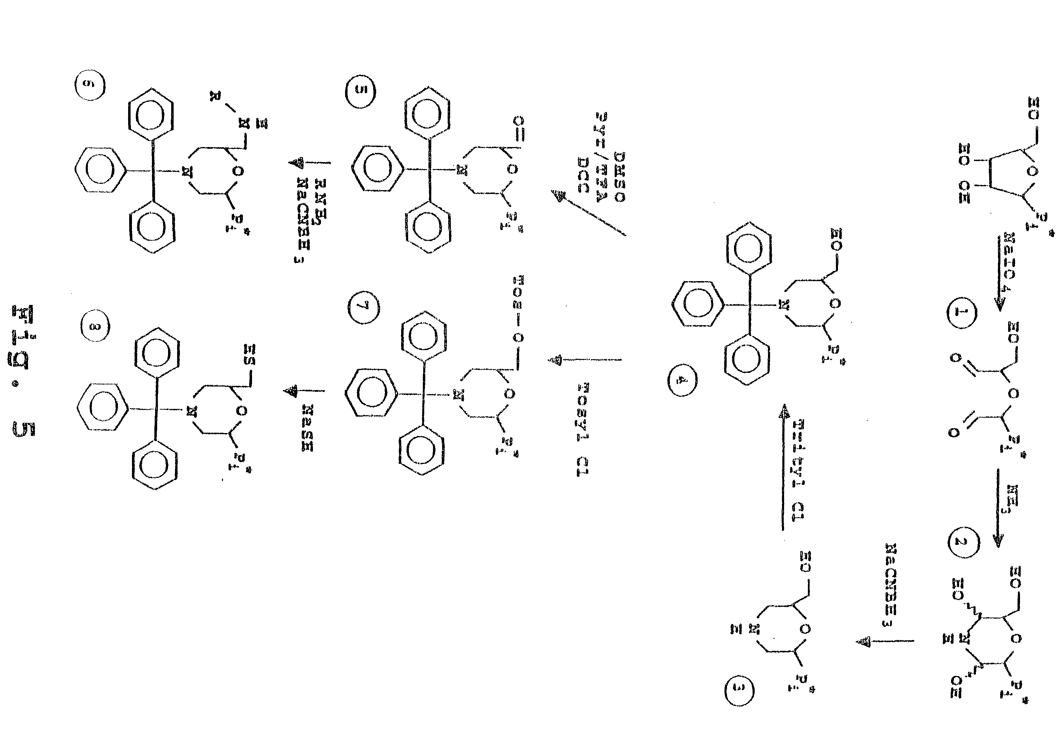

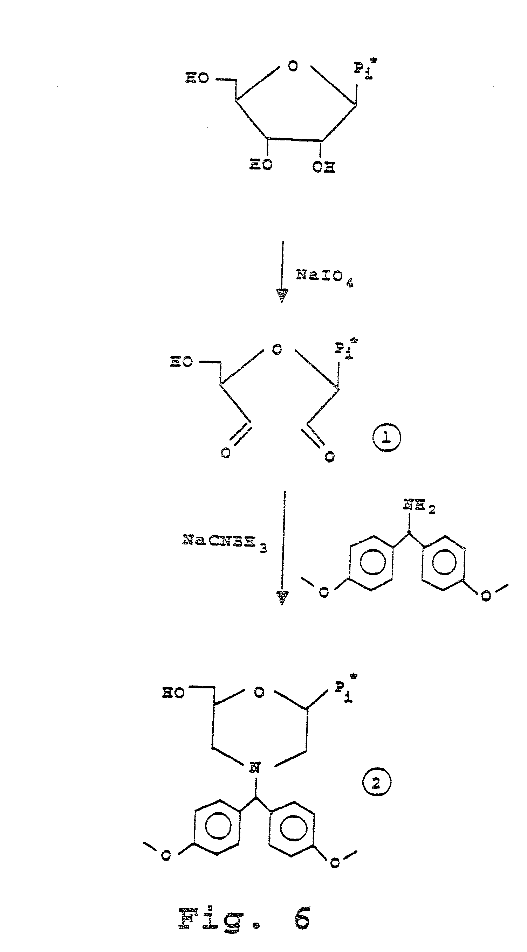

The syntheses of representative morpholino subunits are described particularly in Examples 2-7. With reference to the synthesis scheme depicted in Figure 5, a base-protected ribonucleoside is reacted with sodium periodate to form a transient 2', 3'-dialdehyde which then closes upon ammonia to form a morpholino-ring having 2' and 3' hydroxyl groups (numbered as in the parent ribose, see Figure 1). The compound is then treated with sodium cyanoborohydride to reduce the ring hydroxyl groups. The ring nitrogen is preferably protected by trityl derivatization or by a benzhydraloxycarbonyl group for subsequent subunit coupling. The protective group can be added by reacting the morpholino subunit with trityl chloride or with nitrophenyl benzhydrl carbonate or by reacting the dialdehyde with a primary amine, as illustrated in Figure 6 and described in Example 3. The stereochemistry of the nucleoside starting material is retained as long as the pH of the reaction mixture at the iminium stage is not allowed to go above about 10 or below about 4.

The above synthesis results in a morpholino-ring with an available 5'-hydroxyl. The 5'-hydroxyl can be converted to other active groups including 5' amine and sulfhydral (Example 4).

In the above morpholino synthesis a variety of nitrogen sources can be used -- including particularly ammonia, ammonium hydroxide, ammonium carbonate, and ammonium bicarbonate. Best results are obtained when the reaction solution is maintained near neutrality during the oxidation and morpholino ring closure reactions. This can be accomplished by continually titrating the reaction mix or, more conveniently, by using ammonium biborate as the ammonia source. When the solution is too acidic the yield of product is low and when it is too basic, side products (possibly due to epimerization of the 1' and/or 4' carbons) are produced which are difficult to separate from the desired product. The reducing agent can be added before, during, or after the oxidation step with little noticeable effect on product yield.

Ribonucleosides lacking base protection groups can also be successfully oxidized, ring closed, and reduced in aqueous solution to generate the morpholino ring. However, without base protection the number and quantity of undesired side products frequently increases, particularly in the case of cytidine.

The subunits formed by the above methods contain a 5'-OH, SH, or amine which is modified, reacted with, and/or activated, as described below, to be suitable for coupling to a second morpholino subunit.

### C. Activation and Coupling Reactions

The subunits prepared as above are coupled, in a controlled, sequential manner, often by activating the 5'hydroxyl of one subunit (having a protected morpholino nitrogen) and contacting this activated subunit with another subunit having an unprotected morpholino nitrogen as described in Example 5. It will be recognized that different types of linkages, such as those illustrated below, may be employed in the construction of a single polymer.

The simplest morpholino-type binding polymers are carbamate-linked polymers where the morpholino nitrogen is linked through a carbonyl to the 5'oxygen of another subunit. Experiments conducted in support of the present invention demonstrate that such a polymer effectively binds to a single-stranded DNA target sequence. However, in binding studies with an RNA target, the polymer exhibited unusual binding, as evidenced by a highly atypical hypochromicity profile in the 320 to 230 nm spectral range and lack of a normal thermal denaturation.

Early modeling studies indicated that in a carbamate-linked polymer bound to DNA existing in a B conformation, the backbone of the polymer provides adequate length for binding and the carbamate moieties of the polymer backbone can assume a nearly planar conformation. This modeling result was in good accord with the effective binding of the carbamate-linked polmers to DNA. In contrast, similar modeling studies suggested that binding of the carbamate-linked polymer to an RNA target requires one of the following: (i) the carbamate linkage of the polymer adopt a substantially nonplanar conformation, or (ii) the RNA target sequence adopt a strained conformation in which base-stacking interactions are quite different from that in a normal A conformation. This observation may explain the atypical binding of a carbamate-linked polymer to an RNA target sequence.

The modeling work further indicated that replacing the carbonyl intersubunit linking moiety with either an achiral sulfonyl-containing intersubunit linkage or with a chiral phosphorous-containing linkage would provide added length of about 0.32 angstrom per intersubunit linkage. Such linkages would also provide greater rotational freedom about key bonds, and bond angles of the intersubunit linkage compatible with an oligomer backbone conformation suitable for pairing to both RNA and DNA target sequences in their standard conformations.

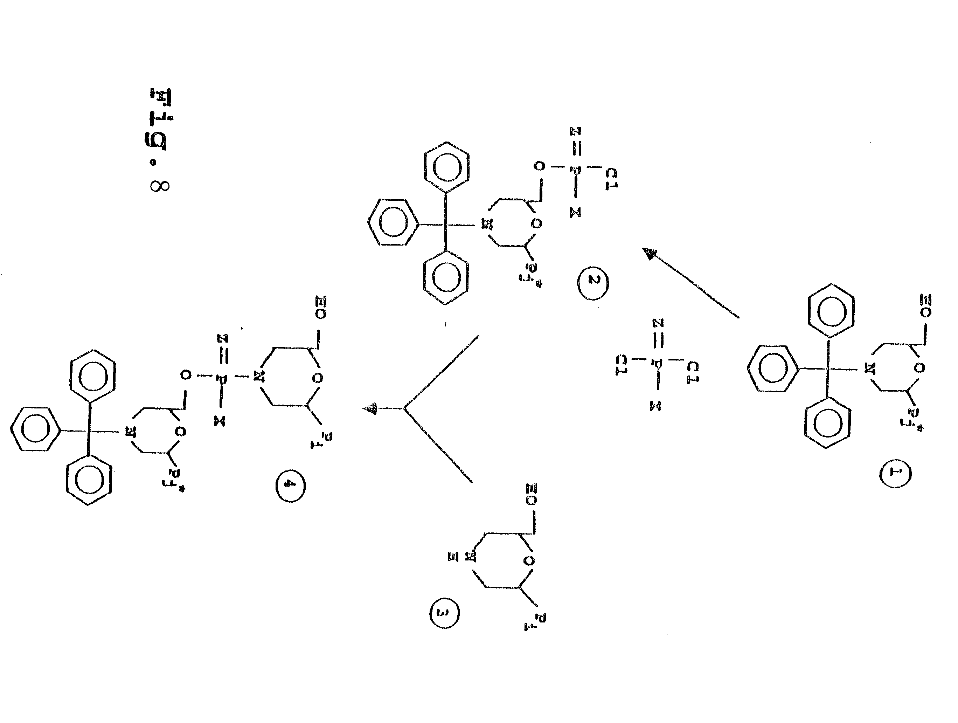

The phosphoramide linkage in the polymers of the invention, shown in Figure 4 (6-atom unit-length backbone) can be formed according to the reaction schemes shown in Figure 8 and detailed in Example 5. The 5'hydroxyl of a protected subunit (Structure 4 of Figure 5) is reacted with a suitable phosphorous-containing compound, such as dichloro-N,N-dimethylamino phosphate, resulting in an activated subunit. The subunit is then reacted with a second subunit having an unprotected morpholino ring nitrogen. A large number of variations are possible in the pendant X moiety and, as described in Example 5, the identity of the X moiety affects the ease of activation and coupling, the stability of the resulting linkage, and, to some extent, target-binding affinity.

In this synthesis the P=O group is essentially interchangeable with the P=S group; reactions with one *are* generally applicable to the other.

An alternative method for forming the phosphoramide linkages is to use carbodiimide coupling: an exemplary, carbodiimide is dicyclohexylcarbodiimide (DCC). Carbodiimide coupling is described in Example 5. By exploiting an observation of Smith et al. (1958), the carbodiimide reagent can also be used to: (a) add a phosphorous (or thiophosphorous) linking moiety to a subunit; or (b) attach a pendant X moiety to a phosphorous (or thiophosphorous) linking moiety.

Additional phosphoramide linkages can be formed by converting the 5'hydroxyl to other functional groups (e.g., SH, CH₂, NR) before activating and coupling the subunits into polymers.

### D. Assembly of Polymers

After selecting a desired polymer length and recognition moiety sequence (guidelines for this are presented below), the polymer is assembled using the general procedures described above. One method of polymer assembly involves initial preparation of an appropriate set of oligomer blocks and then joining these blocks to form the final length polymer. The oligomer blocks used in polymer synthesis need not be of equal size. These blocks are prepared in solution, substantially according to the coupling methods described with reference to Examples 6 and 7. Example 7 outlines such a block synthesis to form a pentamer.

A particular merit of this block assembly method is that any coupling failures in assembly of the full length polymer generates failure sequences which are easily separated from the desired polymer. Example 6 details the assembly of a simple homopolymer by this method. Example 7 describes preparation of a block suitable for use in a heteropolymer. Examples 8 and 9 describe assembly of blocks to form a biologically active heteropolymer. Thus, Examples 7, 8, and 9 together describe a synthetic approach which uses a combination of assembly methods where oligomer blocks are assembled stepwise in solution and then those oligomers joined into full-length polymers.

Typically, the polymer of the invention is attached to a solid support. The polymers may be synthesized by stepwise subunit addition on a solid support, as described in Example 10.

Typically, a solid support, such as glass beads derivatized with acid-stable, long-chain cleavable linkers, are employed as the support material for solid-phase syntheses, and prepared for attachment of the first subunit, or block of subunits, as described in Examples 9 and 10. The glass beads are reacted with a subunit which generally has a readily cleavable protective group on a nitrogen. Whether the morpholino subunit is linked to the support via its morpholino nitrogen or a group at the 5' position depends on the direction of polymer synthesis, ie. to which group the next subunit will be attached.

After coupling the second subunit (or oligomer which may be assembled in solution) to the support, any unreacted nucleophilic sites can be capped by addition of a suitable capping reagent, such as p-nitrophenyl acetate or acetic anhydride, and thereafter the support is washed. The protecting group on the nitrogen of the terminal subunit is removed, typically by acid treatment, and after neutralization, the support is reacted with an excess of the next-in-sequence subunit (or polymer unit) which is activated by one of the methods outlined above. One feature of the solid support assembly method employing stepwise additions of monomeric subunits is the need for high coupling efficiencies at each subunit addition step. This high coupling efficiency is generally achieved by addition of an excess of the activated subunit which maximizes the number of support-bound chains which are chain-elongated.

Chain elongation is continued in this manner, with optional capping of failure sequences after each subunit addition, until the polymer of the desired length and sequence is achieved.

After addition of the final subunit, the terminal backbone moiety may be reacted with a suitable charged or uncharged group, as described in Example 6. The polymer is then cleaved from the support, e.g., by treatment with either ammonium hydroxide or a non-nucleophilic base suitable for effecting β-elimination in the linker joining the polymer to the support. The bases are deprotected and the polymer is purified as described below and in Examples 6, 8, 9, and 10.

### E. Polymer Processing and Purification

Binding polymers assembled in solution (Examples 6 and 8) are typically base-deprotected by suspending in DMSO or DMF and layering on the suspension an equal volume of concentrated ammonium hydroxide. The preparation capped and then mixed with shaking and incubated at 30°C for 16 hrs. Workup includes removing the ammonia under reduced pressure. If a protective group (generally trityl or a related acid-labile moiety) is present, this group is cleaved and the crude polymer preparation is suspended in the appropriate buffer for purification.

Binding polymers assembled by a solid-phase method (Examples 9 and 10) wherein they are linked to the support via an ester linkage can be cleaved from the support by suspending the dried support in DMSO, layering on an equal volume of concentrated NH₄OH, capping tightly, and slowly agitating for 16 hrs at 30°C. The solid support material is removed by filtration and the filtrate is treated as described above.

Alternatively, binding polymers linked to the support via a β-elimination-sensitive linker can be cleaved from the support using a strong nonnucleophilic base 1,8 diazubicyclo(5.4.0.)undec-7-ene (DBU) in DMF. Using this approach one can release the polymer with its bases still protected and thus the polymer is suitable for further modification and/or structural confirmation via fast atom bombardment mass spectroscopy.

Purification of the base-deprotected polymer is preferably carried out at pH 2.5 or pH 11, depending on the pK of the base moieties in the polymer. At pH 2.5 cytosine, adenine, and 2,6-diaminopurine moieties carry a positive charge and guanine carries a partial positive charge. At pH 11 guanine, uracil and hypoxanthine carry a negative charge. For polymers in which about 30% or more of the base-pairing moieties are ionized at pH 2.5, the purification can be carried out by cation exchange on a column of S-Sepharose fast-flow (Pharmacia) developed with a shallow NaCl gradient buffered at pH 2.5. The effluent is monitored at 254 nm and collected in a fraction collector. The full length polymer, which elutes after the shorter failure sequences, can be further purified and desalted on a column of chromatographic grade polypropylene (Polysciences Inc.), eluted with an aqueous gradient of acetonitrile or methanol adjusted to pH 2.5 with formic acid, with the eluant being monitored at 254 nm. The fractions containing the pure product are neutralized and dried under reduced pressure. Salts may be discarded by dissolving the polymer in trifluoroethanol, filtering, and evaporating the trifluoroethanol.

For polymers in which about 30% or more of the base-pairing moieties are ionized at pH 11, the purification may be performed on an anion exchange column of Q Sepharose fast-flow (Pharmacia) developed with an aqueous pH 11 gradient of NaCl. The full-length polymer, which elutes after shorter failure sequences, is further purified and desalted on a polypropylene column eluted with an aqueous pH 11 gradient of acetonitrile. Fractions containing the pure product are processed as above.

The purification methods described above should be carried out so that polymers containing adenine base-pairing moieties are not exposed to pH 11 for more than a few hours at room temperature, to avoid potential base lability problems. The details of the purification methods are outlined in Examples 6, 8, and 9.

In neutral, aqueous solution these morpholino polymers may have a lower solubility than desired. Accordingly, the polymer of the invention typically further includes a moiety at one a both termini which is effective to enhance the solubility of the said polymer in aqueons medium. Typically, the moiety at one or both termini is polyethylene glycol. For most of the polymer types disclosed herein, the addition of said moiety can be accomplished by cleaving the terminal backbone protective group from the completed polymer, and reacting the polymer, with the bases still in the protected state, with excess of carbonyldiimidazole- activated polyethylene glycol (PEG). Thereafter the binding polymer is treated with ammonium hydroxide to remove the base-protected groups, and the polymer is purified as above. The level of solubilization is easily adjusted through proper selection of the PEG material. Suitable PEG fractions having average molecular weights of 200, 400, 600, 1,000, 1,540, 3,400, 4,000, 6,000, 7,500, and 18,500 daltons are commercially available (e.g., Polysciences, Inc.) with PEG1000 often providing the best solubilization. The solubilizing moiety may be linked to the polymer through a cleavable linkage, if desired, to allow the polymer to be released from the solubilizing agent, e.g., by esterase or peptidase enzymes.

It will be appreciated that the polymer may be further derivatized or labeled according to known procedures. For example, the polymer may be radiolabeled by preparing the polymer subunits from radiolabeled ribonucleosides or by attaching a radiolabeled amino acid at one terminus. The polymer may be readily derivatized, e.g., employing modifications of the above subunit coupling reactions, with enzymes, chromophoric groups, or the like, where the polymer is to be used as a diagnostic probe. Further, the polymer may be derivatized with biomolecules which serve to target the polymers to specific tissues or cell types.

### F. Structural Characterization

Fully-protected binding polymers of moderate size (10 to 20 subunits) often give a strong molecular ion in FAB (Fast Atom Bombardment) mass spectroscopy, providing a key confirmation of the polymer length.

Further, COSY-NMR (two-dimensional correlated spectroscopy) of the deprotected and purified polymer provides information on the ratio of the different base-pairing moieties in the polymer as well as quantitative information on the ratio of binding polymer to any solubilizing or other type moiety which may have been linked thereto.

Mobilities on ion exchange columns also provide information on the number of C + A base-pairing moieties in a polymer when purification is carried out at pH 2.5 and information on the number of G + U residues when the purification is run at pH 11. Structural verification is easiest when the polymers have been assembled from oligomer blocks, such as in Examples 6, 8, and 9, since any failure sequences then differ more substantially from the full-length sequences.

The UV profiles of the polymers at pH 1, 7, and 13 can provide information about the relative nucleotide composition of the polymer.

Assessment of a morpholino-based polymer's affinity for its target sequence is carried out by examining the melting curve of the polymer/target duplex, as illustrated in Example 6. Further, comparisons can be made between the melting curve of a regular nucleic acid duplex (such as p(dC)₆/p(dG)₆) and the melting curve of a hybrid duplex containing a corresponding morpholino-based polymer (such as (morpholino C)₆/p(dG)₆).

The above characterization steps have been applied to a morpholino-based phosphordiamidate-linked poly(C) hexamer where Y is oxygen, X is N(CH₃)₂, and Z is oxygen, as described in Example 6. Characterization of the full-length oligomer was achieved by proton NMR and negative ion FAB mass spectroscopy. With these morpholino oligomers, the fragmentation of the oligomers is greatly suppressed so that little sequence information is available. However, the parent ion signal is quite strong and allows confirmation of the composition of the morpholino oligomer (see Example 6). In order to increase water solubility a polyethylene glycol (PEG) tail was attached to the oligomers. 5 equivalents of PEG 1000 was treated with one equivalent of bis(p-nitrophenyl)carbonate to give monoactivated PEG. Detritylation of the hexamer with 1% acetic acid in trifluoroethanol afforded a free morpholino ring nitrogen. Treatment of the hexamer containing the free amine with activated PEG1000 under standard coupling conditions resulted in attachment of the PEG tail to the hexamer. The bases were deprotected by treatment of the tailed hexamer with concentrated ammonia for 24 hours. The tailed hexamer was taken up in pH 2.5 buffer and purified by cation exchange chromatography on S-Sepharose Fast Flow™ eluted with a potassium chloride gradient. After neutralization the eluant was desalted on a polypropylene column eluted with a water/acetonitrile gradient. The tailed hexamer was found to be freely soluble in pH 7.5 buffer.

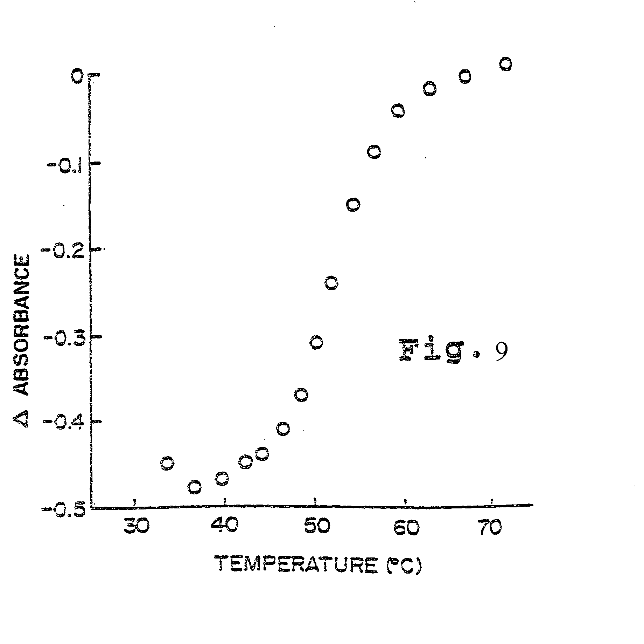

The stability of complexes of the tailed hexamer with complementary nucleic acids was investigated by thermal denaturation experiments. Difference spectra between mixed and unmixed samples of the tailed hexamer and the selected phosphodiester complement were obtained from 14°C to 85°C and over the 320 to 260 nm range (see Example 6). At 60 micromolar in C monomer and 60 micromolar in G monomer the difference UV spectrum of the tailed hexamer, (morphC)₆ with poly (dG) gave a Tₘ value of 79°C. The corresponding (morphC)₆ with poly(G) gave a Tm value of 51.5°C (see Example 6 and Figure 9).

### G. Diagnostic Applications

The target-specific polymers of the invention can be used in a variety of diagnostic assays for detection of RNA or DNA having a given target sequence. Accordingly, the present invention provides a method for detecting, in a sample, the presence of a polynucleotide having a selected target sequence, comprising contacting a polymer of the invention with said polynucleotide, where said polymer (i) has a series of base-pairing moieties capable of binding to the selected target sequence, and (ii) is labeled with a detectable reporter group, and where said reacting of the polymer with the polynucleotide is carried out under conditions effective to allow formation of a hybridization complex between the polymer and the target sequence, and detecting the presence of the reporter group.

In one general application, the polymers are labeled with a suitable radiolabel or other detectable reporter group. Target polynucleotide, typically a single stranded polynucleotide such as DNA or RNA, which can be bound to a solid support, is reacted with the polymer under hybridization conditions, allowed to anneal, and then the sample is examined for the presence of polymer reporter group.

The diagnostic assay can be carried out according to standard procedures, with suitable adjustment of the hybridization conditions to allow polymer hybridization with the target region. Further, the polymer can be designed for hybridization with the target at a higher melting temperature than the complementary polynucleotide strand, since polymer binding does not entail backbone charge repulsion effects. Therefore, the polymer can bind to the target at a temperature above the normal polynucleotide melting temperature: this is one advantage of the polymer over conventional oligonucleotide probes. This binding at elevated temperature minimizes the problem of competition for binding to the target between the probe and any corresponding single-strand oligonucleotide which may be present in the diagnostic mixture.

In a second general type of diagnostic application, the polymers are linked to a solid support, for capture of target RNA or DNA to the support. Thus, the present invention also provides a method of isolating, from a sample, a nucleic acid having a selected target sequence, comprising
contacting a polymer of the invention with said nucleic acid, where said polymer (i) has a series of base-pairing moieties capable of binding to the selected target sequence, and (ii) is attached to a solid support, and (iii) where said contacting of the polymer with the nucleic acid is carried out under conditions effective to allow formation of a hybridization complex between the polymer and the target sequence, and
isolating said nucleic acid.

The solid support, e.g., polymeric microparticles, can be prepared by linking the polymers to the support according to the methods described above or by conventional derivatization procedures. Alternatively, where the polymers are synthesized on a solid support this support may also serve as the assay support.

According to one feature of this assay system, the target polynucleotide molecules which are captured on the support by base-specific binding to the polymers can be detected on the basis of their backbone charge, since the support-bound polymers are themselves substantially uncharged. To this end, the assay system may also include polycationic reporter molecules which are designed to bind to the fully charged analyte backbone, but not the uncharged (or substantially uncharged) polymer backbone, under selected binding conditions.

In one embodiment the reporter molecules are composed of a polycationic moiety or tail designed to bind electrostatically to a fully charged polynucleotide, under conditions where the reporter does not bind to the less charged or uncharged binding polymer carried on the diagnostic reagent; one or more reporter groups may be attached to the tail, adapted to produce a signal by which the presence of the reporter can be detected.
Methods for forming polycationic molecules and for attaching reporter molecules to cationic compounds are known.

Each reporter molecule carries one or more reporter groups, and each polynucleotide can accommodate binding of typically several thousand or more reporter molecules. Thus the system has an amplification factor, in terms of reporter signal per bound analyte molecule, of several orders of magnitude. In addition, the method has the advantage, noted above, that the polynucleotide binding reaction can be carried out under conditions in which binding competition with complementary nucleotide strands does not occur.

The design considerations applied in preparing a polynucleotide binding polymer for use as a diagnostic reagent are governed by the nature of the target analyte and the reaction conditions under which the analyte is to be assayed. As a first consideration, there is selected a non-homopolymeric target base sequence against which the polymer is directed. This target sequence is generally single-stranded and preferably unique to the analyte being assayed.

The probability of occurrence of a given n-base target sequence is approximately (1/4)ⁿ. Accordingly, a given n-base target sequence would be expected to occur approximately once in a polymer containing 4ⁿ bases. Therefore, the probability P that a given n-base sequence will occur in polynucleotides containing a total of N unique-sequence bases is approximately P=N/4ⁿ. To illustrate, the probability P that a 9-base target sequence will be found in a 20 kilobase polynucleotide is about 20x10³/2x10⁵ or 0.08, the probability that a 16-base target sequence will be present is about 20x10³/4.3x10⁹ or 0.0000047. From these calculations, it can be seen that a polymer having 9-16 recognition moieties specific for a defined 9-16 base target sequence should have high specificity for the target sequence in an assay mixture containing only viral genomes, whose greatest complexities correspond to about 400K of unique-sequence bases.

Similar calculations show that a 12 to 16 subunit polymer can provide adequate specificity for a viral or bacterial target sequence in an assay mixture containing viral and bacterial genomic material only; largest genomic sizes about 5,000 kilobases. A 16 to 22 subunit polymer can provide adequate specificity for a target sequence in a polynucleotide mixture containing mammalian genomic DNA material; genomic sizes of about 5 billion base pairs of unique-sequence DNA.

The polymer/analyte binding affinity, and particularly the temperature at which the polymer just binds with the target sequence (the melting temperature, or Tm) can be selectively varied according to the following criteria: (a) number of subunits in the polymer; (b) the number of hydrogen bonds that can be formed between the base-pairing moieties and the corresponding, complementary bases of the analyte target sequence; (c) unit length of the polymer backbone; (d) the particular intersubunit linkages; and (e) concentration of denaturants, such as formamide, which reduces the temperature of melting.

From a number of studies on model nucleic acid duplexes it is known that the melting temperature of oligonucleotide duplexes in the 10 to 20 bp range increases roughly 3°C per additional base pair formed by two hydrogen bonds, and about 6°C per additional base pair formed by three hydrogen bonds. Therefore, the target sequence length originally selected to insure high binding specificity with the polymer may be extended to achieve a desired melting temperature under selected assay conditions.

Also, where the recognition moieties used in constructing the polymer are the standard nucleic acid bases the target sequence may be selected to have a high percentage of guanine plus cytosine bases to achieve a relatively high polymer/analyte melting temperature. On the other hand, to achieve a lower melting temperature a target sequence is selected which contains a relatively high percentage of adenine plus thymine bases.

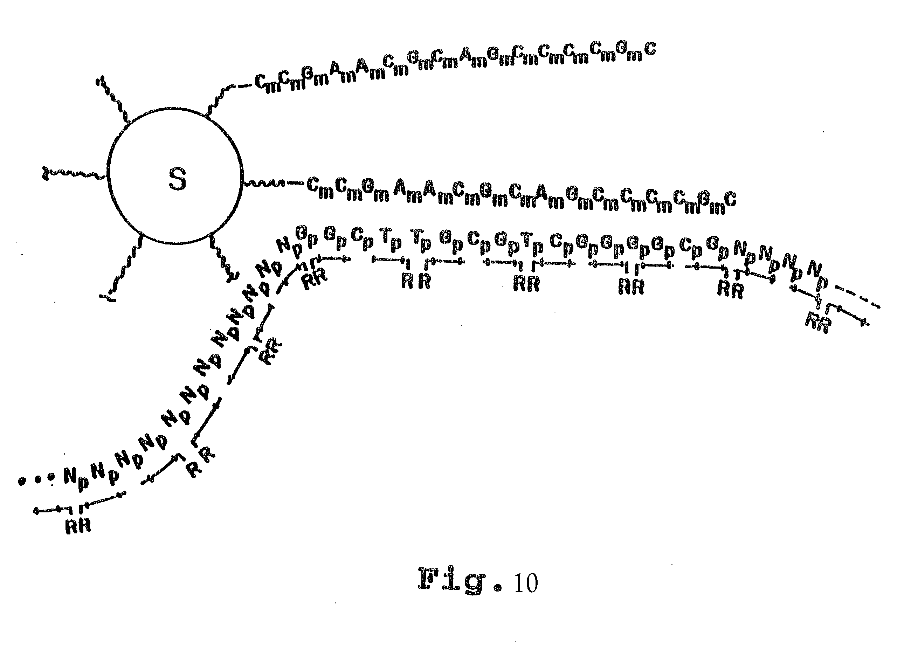

The binding components in the diagnostic system, as they function in the solid-support diagnostic method just described, are illustrated in Figure 10. Here "S", the assay reagent, is the solid support having a number of binding polymers attached to its surface through spacer arms indicated by sawtooth lines. In the assay procedure, the target DNA in single strand form is reacted with the support-bound polymers under hybridization conditions, and the solid support is then washed to remove non-hybridized nucleic acid material.

The washed support is then reacted with the reporter, under conditions which favor electrostatic binding of the reporter cationic moiety to the target DNA backbone. The reporter shown in Figure 10 is a dicationic molecule having a reporter group R.

After reaction with the reporter solution, typically at room temperature for a few seconds, the reagent is washed to remove unbound reporter, and then the assay reagent is assessed for bound reporter. One approach in determining the amount of reporter associated with the reagent, particularly in the case of fluorescent or chromophoric reporter groups, is to elute the reporter from the reagent with a high salt solution and then assess the eluate for reporter.

The polymer of the invention can undergo sequence-specific binding to duplex nucleic acids, via base-pair-specific hydrogen bonding sites which are accessible through the major groove of the double helix. This bonding can occur in a duplex region in which at least 70% of the bases on one strand are purines and a corresponding percent of the bases on the other strand are pyrimidines. The duplex binding polymer preferably includes 2,6-diaminopurine hydrogen bonding moieties for binding to T-A or U-A base pairs, and guanine hydrogen-bonding moieties for binding to C-G base pairs as illustrated in Figure 7. Thus, for these special target sequences, the polymer of the invention can be used for diagnostic assays of the types just described, but where the target nucleic acid is in nondenatured, duplex form.

### H. Other Applications

The polymers of the instant invention can be used in place of standard RNA or DNA oligomers for a number of standard laboratory procedures. As mentioned above, morpholino-based polymers can be fixed to a solid support and used to isolate complementary nucleic acid sequences, for example, purification of a specific mRNA from a poly-A fraction (Goldberg et al). The instant polymers are advantageous for such applications since they are inexpensive and straightforward to prepare from activated subunits.

A large number of applications in molecular biology can be found for labeled morpholino-based polymers. Morpholino-based polymers can be easily and efficiently end-labelled by the inclusion in the last step of the polymer synthesis an activated and labelled morpholino-based subunit or, preferably, an ³⁵S-labelled methionine, as indicated above. The type of label to be used is dependent on the final application of the polymer; such labels include radioactive (³H, ¹⁴C, ³²P, or ³⁵S) nucleosides or amino acids, or biotin. Labelled morpholino-based oligonucleotide analogs can act as efficient probes in, for example, colony hybridization (Grunstein et al), RNA hybridizations (Thomas), DNA hybridizations (Southern), and gene bank screening (Szostak et al).

The polymers of the invention also have potential use as "antisense" therapeutic agents. A number of uncharged nucleic acid analogs, which are nearly isostructural with DNA, have been used as anti-viral and anti-cancer agents. Morpholino-based polymers of the present invention have been tested *in vitro* against HIV-I (Human Immunodeficiency Virus). A 16-mer complementary to the primer binding site of HIV-I (Myers et al.) has been shown to reduce synthesis of the viral P24 protein by approximately 35% (Examples 8 and 9). In corresponding uninfected cells the morpholino-based polymers caused no apparent cell toxicity.

The polymers of the present invention have also been tested *in vivo* against HSV-I (Herpes Simplex Virus I) ocular infection in mice (Example 10). A 12-mer complementary to a splice junction site in HSV-I was tested for its ability to protect mice ocularly infected with HSV-I. Untreated mice showed a survival rate of 65% at 5 days post-infection, whereas mice treated with a topical ointment containing the morpholino-based polymer had a survival rate of 90%.

In addition to the above described *in vitro* and *in vivo* anti-viral properties of the morpholino-based polymers, the polymers of the present invention provide several advantages over more conventional anti-sense agents.

First, the morpholino polymers are less expensive to synthesize than deoxyribonucleoside-derived polymers. This is due in part to the fact that the morpholino subunits used in polymer synthesis are derived from ribonucleosides, rather than the rarer and much more expensive deoxyribonucleosides. Also, as noted above, the coupling reaction between a phosphorous and an amine of a second subunit occurs under relatively mild conditions, so that protection steps and other precautions needed to avoid unwanted reactions are simplified. This is in contrast to standard ribo- and deoxyribonucleotide polymer synthesis where coupling through a phosphate ester linkage requires that the coupling reagents be highly reactive and that the reaction be carried out under stringent reaction/protection conditions. This advantage in polymer synthesis also applies, of course, to diagnostic uses of the polymer.

Second, polymer binding to its target may give better target inactivation than with DNA-type antisense agents, since the morpholino polymer/target duplex is not susceptible to duplex unwinding mechanisms in the cell.

Third, the morpholino-based polymer is also more stable within the cell than DNA, RNA, and their thiophosphate-linked analogs because the morpholino polymer backbone linkages are not susceptible to cleavage by cellular nucleases.

Fourth, in therapeutic applications involving cellular uptake of the compound, the uncharged morpholino polymer enters cells much more efficiently than a charged oligonucleotide.

In the context of therapeutic applications, the morpholino polymers of the present invention may be targeted against double-stranded genetic sequences in which one strand contains predominantly purines and the other strand contains predominantly pyrimidines, as illustrated in Figure 7.

Further, when a messenger RNA is coded by the mostly purine strand of the duplex target sequence, morpholino binding polymers targeted to the duplex have potential for also inactivating the mRNA. Thus such a polymer has the potential for inactivating key genetic sequences of a pathogen in both single-stranded and double-stranded forms.

In 1981 it was reported that short (3 to 7 subunits) methylphosphonate-linked DNA analogs complementary to portions of the Shine-Dalgarano consensus sequence of procaryotic mRNAs were effective in disrupting bacterial protein synthesis in bacterial lysates and in a special permeable strain of bacteria. However, such agents failed to inhibit protein synthesis in normal bacteria (Jayaramon, 1981).

Experiments performed in support of the instant invention show that polymers of 3 to 5 subunits in length can be effective to block protein synthesis in normal bacteria by using a combination of bases which result in a high target-binding affinity. More specifically, the following oligomers and oligomer combinations can perturb protein synthesis in normal intact bacteria (where D is 2,6-Diaminopurine or Adenine; G is Guanine; B is 5-Bromouracil, other 5-Halouracil or Uracil; and sequences are shown with their 5' end to the left): DGG, BDDG, DDGG; DGGD; GGDG; GDGG; DGGB; GGBG; GGAGG; GGDGG; and the combinations BDD + GGDG; DDG + GDGG; DGG + DGGB; GGD + GGBG; BDDG + GDG; DDGG + DGG; DGGD + GGB; GGDG + GBG; BDD + GGDG + GBG.

The use of short binding-enhanced oligomers to disrupt the biological activity of an RNA sequence which plays a key role in the metabolism of a target class of organisms but not a correspondingly important role in higher organisms should be broadly adaptable to a variety of pathogenic organisms (e.g., bacteria and fungi) having a cell wall which excludes the entrance of longer polymers.

The following examples illustrate methods of subunit and polymer synthesis, and uses of the polymer composition of the invention.

### Example 1

### Base Protection of Ribonucleosides

The following ribonucleosides are obtained from Sigma Chemical Co. (St. Louis, MO): uridine, guanosine, 5-methyluridine, adenosine, cytidine, 5-bromouridine, and inosine.

2, 6-diamino-9- (B-D-ribofuranosyl) -9H-purine (2, 6-diaminopurine riboside) is obtained from Pfaltz and Bauer, Inc., Division of Aceto Chemical Co., Inc. (Waterbury, CT). The following nucleosides are prepared by the literature methods indicated:
1-β-D-ribofuranosyl)-2-pyrimidinone (2-hydroxypyrimidine riboside) is prepared by the procedure of Niedballa.
2-amino-9-β-D-ribofuranosyl)-1,6-dihydro-6hpurine-6 -thione (thioguanosine) is prepared by the procedure of Fox. Dimethoxytrityl chloride, N6-benzoyladenosine, N4-benzoylcytidine, and N2-benzoylguanosine are obtained from Sigma Chemicals. 9-fluorenylmethoxycarbonyl chloride (FMOC chloride), trimethylchlorosilane, isobutyric anhydride, 4-nitrobenzoyl chloride, naphthalic anhydride, and all organic solvents for reactions and chromatography were obtained from Aldrich Chemical Co. (Milwaukee, WI). Silica Gel is obtained from EM Science (Cherry Hill, NJ).

When activation of the subunits is achieved using dihalogenated electrophiles (e.g., P(O)Cl₂N(CH₃)₂ or P(S)Cl₂N(CH₃)₂), better yields of activated subunits are often obtained by using protective groups which leave no acidic protons on the purine and pyrimidine exocyclic amines. Examples of such exocyclic amine moieties are as follows: the N6 of adenine, the N4 of cytosine, the N2 of guanine, and the N2 and N6 of diaminopurine. Suitable protective groups for this purpose include the naphthaloyl group (Dikshit) and the amidine groups developed by McBride et al (1986). In addition, use of dihalogenated electrophiles for subunit activation generally gives better results when the O6 of guanine moieties is protected; this protection is achieved using the p-nitrophenethyl group (Trichtinger).

### Guanosine

In order to minimize side reactions during subunit activations it is often desirable to protect the guanine moity on both the N2 and O6 using the procedure of Trichtinger et al. (1983).

The N-2 9-fluorenylmethoxycarbonyl derivative of guanosine is prepared by the procedure below which is general for the protection of nucleoside amino groups: guanosine (1 mmole) is suspended in pyridine (5 ml) and treated with trimethyl-chlorosilane (5 mmol). After the solution is stirred for 15 minutes, 9-fluorenylmethoxycarbonyl chloride (5 mmol) is added and the solution is maintained at room temperature for 3 hours. The reaction is cooled in an ice bath and water (1 ml) is added. After stirring for 5 minutes conc. ammonia (1 ml) is added, and the reaction is stirred for 15 minutes. The solution is evaporated to near dryness and the residue is dissolved in chloroform (10 ml). This solution is washed with sodium bicarbonate solution (5 ml, 10%), dried over sodium sulfate and evaporated. The residue is coevaporated several times with toluene and the product chromatographed on silica gel using a gradient of methanol in methylene chloride (0-50%).

N-2-Isobutyrylguanosine is prepared by the method of Letsinger. Further protection of the O6 with a nitrophenethyl moiety is often desirable and can be carried out by several methods (Gait, 1984).

N-2-acetylguanosine is obtained by the method of Reese. N-2-naphthaloylguanosine is prepared by the method of Dikshit; this reference provides a general method for the protection of nucleoside amine groups.

### Adenosine

The N-6 2-(4-nitrophenyl)-ethoxycarbonyl derivative is prepared by the method of Himmelsbach.

N-6 (4-nitrobenzoyl)adenosine is prepared using the procedure above for FMOC-guanosine except that 4-nitrobenzoyl chloride is substituted for FMOC chloride.

The N-6 2-(phenylsulfonyl)-ethoxycarbonyl derivative is prepared by the procedure for FMOC guanosine except the 2-(phenylsulfonyl)-ethyl chloroformate (Balgobin) is used as the acylating agent and N-methylimidazole or pyridine is used as the solvent.

N-6 naphthoyladenosine is prepared by the method of Dikshit; this reference provides a general method for the protection of nucleoside amine groups.

### 2,6-diaminopurine riboside

The N-2,N-6-bis(9-fluorenylmethoxycarbonyl) derivative of 2,6-diaminopurine riboside is prepared by the general procedure described for guanosine.

The N-2,N-6-bis(isobutyryl) derivative is prepared by the general procedure described for guanosine.

### Thioguanosine

The N-2(9-fluorenylmethoxycarbonyl) derivative of thioguanosine is prepared by the general procedure described for guanosine.

### Uridine

To minimize undesired side products during the subunit activation step it is sometimes desirable to protect the N3 of the uracil moiety. 5' O-tritylated uridine-2',3'-acetonide is converted to the N3 anisoyl derivative by the procedure of Kamimura et al. (1983). The product is then treated with hot 80% acetic acid or 0.1 N HCl in THF to cleave the protective groups on the ribose moiety.

### Example 2

### Synthesis of 5'-OH Morpholino Subunits

The steps in the method are illustrated in Figure 5, with reference to structures shown in Figure 5.

The base-protected ribonucleoside is oxidized with periodate to a 2'-3' dialdehyde (Structure 1). The dialdehyde is closed on ammonia or primary amine (Structure 2) and the 2' and 3' hydroxyls (numbered as in the parent ribose) are removed by reduction with cyanoborohydride (Structure 3).

An example of this general synthetic scheme is described below with reference to the synthesis of a base-protected cytosine (Pᵢ*) morpholino subunit. To 1.6 1 of methanol is added, with stirring, 0.1 mole of N4-benzoylcytidine and 0.105 mole sodium periodate dissolved in 100 ml of water. After 5 minutes, 0.12 mole of ammonium biborate is added, and the mixture is stirred 1 hour at room temperature, chilled and filtered. To the filtrate is added 0.12 mole sodium cyanoborohydride. After 10 minutes, 0.20 mole of toluenesulfonic acid is added. After another 30 minutes, another 0.20 mole of toluenesulfonic acid is added and the mixture is chilled and filtered. The solid precipitate is washed with two 500 ml portions of water and dried under vacuum to give the tosylate salt of the free amine shown in Structure 3.

The use of a moderately strong (pKa < 3) aromatic acid, such as toluenesulfonic acid or 2-naphthalenesulfonic acid, provides ease of handling,. significantly improved yields, and a high level of product purity.

The base-protected morpholino subunit is then protected at the annular nitrogen of the morpholino ring using trityl chloride or benzyhydral nitrophenyl carbonate (Structure 4). Alternatively, the 5' hydroxyl can be protected with a trialkylsilyl group.

As an example of a protection step, to 2 liters of acetonitrile is added, with stirring, 0.1 mole of the tosylate salt from above followed by 0.26 mole of triethylamine and 0.15 mole of trityl chloride. The mixture is covered and stirred for 1 hour at room temperature after which 100 ml methanol is added, followed by stirring for 15 minutes. After drying by rotovaping, 400 ml of methanol is added. After the solid is thoroughly suspended as a slurry, 5 liters of water is added, the mixture is stirred for 30 minutes and filtered. The solid is washed with 1 liter of water, filtered, and dried under vacuum. The solid is resuspended in 500 ml of dichloromethane, filtered, and rotovaped until precipitation just begins, after which 1 liter of hexane is added and stirred for 15 minutes. The solid is removed by filtering, and dried under vacuum.

The above procedure yields the base-protected morpholino subunit tritylated on the morpholino nitrogen and having a free 5' hydroxyl (Structure 4).

### Example 3

### Alternative Synthesis of Morpholino Subunits

This example describes an alternative preparation of a morpholino subunit containing an acid-labile moiety linked to the morpholino ring nitrogen. The steps are described with respect to Figure 6.

The subunit is prepared by oxidizing a ribonucleoside with periodate, as in Example 2, and closing the resultant dialdehyde (Structure 1) on the primary amine 4,4'-dimethoxybenzhydrylamine (which can be prepared by the method of Greenlee, 1984) buffered with benzotriazole, or p-nitrophenol. Reduction with sodium cyanoborohydride, carried out as in Example 2, gives a morpholino subunit (Structure 2) having a 4,4'-dimethoxybenzhydryl group on the morpholino nitrogen.

One feature of this procedure is that it is particularly useful for preparing morpholino subunits from ribonucleosides which do not have a protective group on the base (e.g., uridine).

### Example 4

### Conversion of 5'Hydroxyl to 5'Amine and to 5'Sulfhydral

The steps in the synthesis are described with reference to structures shown in Figure 5.

### A. Conversion to the Amine

The 5'hydroxyl of the doubly-protected morpholino subunit (Structure 4, Figure 5) can be converted to an amine as follows. To 500 ml of DMSO is added 1.0 mole of pyridine (Pyr), 0.5 mole of triflouroacetic acid (TFA), and 0.1 mole of the morpholino subunit. The mixture is stirred until all components are dissolved and then 0.5 mole of either diisopropylcarbodiimide (DIC) or dicyclohexylcarbodiimide (DCC) is added. After 2 hours the reaction mixture is added to 8 liters of rapidly stirred brine. This solution is stirred for 30 minutes and then filtered. The resultant solid is dried briefly, washed with 1 liter of ice cold hexanes and filtered. The solid is added to 0.2 mole of sodium cyanoborohydride in 1 liter of methanol and stirred for 10 minutes. To this mixture, 0.4 mole of benzotriazole or p-nitrophenol is added, followed the addition of 0.2 mole of methylamine (40% in H₂O). The preparation is stirred four hours at room temperature. [Note: the benzotriazole or p-nitrophenol buffers the reaction mixture to prevent racemization at the 4' carbon of the subunit at the iminium stage of the reductive alkylation.] Finally, the reaction mixture is poured into 5 liters of water, stirred until a good precipitate forms, and the solid (Structure 6, Figure 5) is collected and dried.

### B. Conversion to the Sulfhydral

The 5' hydroxyl of the doubly-protected morpholino subunit is converted to a sulfhydral as follows. One-tenth mole of the 5'-hydroxyl subunit (Structure 4, Figure 5) is added to 1 liter of pyridine, followed by the addition of 0.12 mole of toluenesulfonylchloride. This solution is stirred for 3 hours at room temperature and the reaction yields the product shown as Structure 7 of Figure 5. The pyridine is removed by rotovapping, and to the solid is added 0.5 mole of fresh sodium hydrosulfide in 1 liter of methanol DMF containing NaI. This reaction mixture is stirred at room temperature overnight. The mixture is added to 5 liters of water, stirred 20 minutes, and the resulting solid material is collected by filtration and dried to give the product shown as Structure 8 of Figure 5.

### Example 5

### Activation and Coupling to Give Phosphoramide Linkages

### A. X = -CH₃

Example 5A describes the activation and coupling of a 5'hydroxyl subunit, prepared as in Example 2 or 3, to a second subunit having a free morpholino ring nitrogen to give an alkylphosphonamidate intersubunit linkage. The example is described with reference to structures in Figure 8 where X is an alkyl group.

One mmole of 5'hydroxyl subunit, base-protected and tritylated on the morpholino nitrogen (Structure 4 of Figure 5), is dissolved in 20 ml of dichloromethane. To this solution 4 mmole of N-ethylmorpholine and 1.1 mmole of methylphosphonic dichloride, for Z = O (or methylthiophosphonic dichloride, for Z = S), are added, followed by the addition of 1 mmole of N-methylimidazole. After one hour the reaction solution is washed with aqueous NaH₂PO₄. The activated subunit is isolated by chromatography on silica gel developed with ethyl acetate (Structure 2 of Figure 8 where X is methyl). This activated subunit can be directly linked to the morpholino nitrogen of a second subunit (Structure 3) by mixing them in DMF. This coupling reaction yields the dimer shown as Structure 4 (where X is -CH₃).

An alternative activation and coupling procedure entails dissolving one mmole of 5'hydroxyl subunit, base-protected and tritylated on the morpholino nitrogen (Structure 4 of Figure 5), in 20 ml of dichloromethane. To this solution 4 mmole of N,N-diethylaniline and 0.1 mmole of 4-methoxypyridine-N-oxide are added. After dissolution 1.1 mmole of methylphosphonic dichloride, for Z = O (or methylthiophosphonic dichloride, for Z = S), is added. After two hours the product (Structure 2 of Figure 10, where X is methyl) is isolated by chromatography on silica gel developed with 10% acetone/90% chloroform. One mmole of this activated subunit can be directly linked to the morpholino nitrogen of a second subunit (Structure 3) by mixing them in dichloromethane containing 1.5 mmole of N,N-diisopropylaminoethyl isobutyrate (DIPAEIB) to give the dimer shown as Structure 4 (where X is -CH₃).

The tertiary amine/ester, DIPAEIB, is prepared by mixing 1.1 mole of isobutyryl chloride, 1 mole of N,N-diisopropylaminoethanol, and 1.5 mole of pyridine and heating for 30 min at 110°C. The preparation is washed with 0.5 mole of NaOH in 500 ml H₂O and distilled to give the desired amine/ester boiling at 115°C at 30 mm Hg.

The alkylphosphonamidate intersubunit linkage (Structure 4 where X is -CH₃) is very stable to ammonia used for base deprotections. In contrast, the linkage is sensitive to relatively strong acids. For instance, the linkage has a half time of cleavage of about 3 hours in 2% dichloroacetic acid in dichloromethane. However, the linkage shows no detectable cleavage after 18 hours in 2% acetic acid in trifluoroethanol, conditions suitable for detritylation of the morpholino nitrogen.

### B. X = -O-CH₂CH₃

Example 5B describes the activation and coupling of a 5'hydroxyl subunit, prepared as in Example 2 or 3, to a second subunit having a free morpholino ring nitrogen to give a phosphodiesteramide intersubunit linkage. The example is described with reference to structures in Figure 8 where X is an alkoxide group.

One mmole of 5'hydroxyl subunit, base-protected and tritylated on the morpholino nitrogen (Structure 4 of Figure 5), is suspended in 80 ml of benzene and 2.2 mmole of N-methylimidazole is added. After the subunit is dissolved 1.2 mmole of ethyl dichlorophosphate for Z = O (or ethyldichlorothiophosphate for Z = S) are added. After an hour the reaction solution is washed with aqueous NaH₂PO₄. The activated subunit is isolated by chromatography on silica gel developed with ethyl acetate (Structure 2 in Figure 8, where X is -O-CH₂CH₃). This activated subunit can be directly linked to the morpholino nitrogen of a second subunit (Structure 3) by mixing in DMF. This coupling reaction yields the dimer shown as Structure 4.

When ethyldichlorothiophosphate (Z=S) is used for activation of the subunits, improved yields are obtained with the following modifications. One mmole of 5' hydroxyl subunit, base-protected and tritylated on the morpholino nitrogen (Structure 4 of Figure 5), is suspended in 20 ml of chloroform. To this solution 1 ml of N-methylimidazole is added, followed by the addition of 1.6 ml of ethyldichlorothiophosphate (Aldrich Chem. Co.). After 1 hour the subunit product is purified by silica gel chromatography developed with 20% acetone/80% chloroform. This activated subunit (Structure 2, where X is -O-CH₂-CH₃ and Z is sulfur) can be coupled to the morpholino nitrogen of a second subunit as described above.

An alternative activation and coupling procedure entails dissolving one mmole of 5'hydroxyl subunit, base-protected and tritylated on the morpholino nitrogen (Structure 4 of Figure 5), in 20 ml of dichloromethane. To this solution 4 mmole of N,N-diethylaniline and 0,2 mmole of 4-methoxypyridine-N-oxide are added. After dissolution, 1.1 mmole of ethyldichlorophosphate, for Z = O (or ethyldichlorothiophosphate, for Z = S), is added. After one hour the product (Structure 2 of Figure 8, where X is ethoxy) is isolated by chromatography on silica gel developed with 10% acetone/90% chloroform. One mmole of this activated subunit can be directly linked to the morpholino nitrogen of a second subunit (Structure 3) by mixing them in dichloromethane containing 1,5 mmole of N,N-diisopropylaminoethyl isobutyrate (DIPAEIB) to give the dimer shown as Structure 4 (where X is O-CH₂-CH₃).

### C. X = -F

Example 5C describes the coupling of a 5'hydroxyl subunit, prepared as in Example 2 or 3, to a second subunit having a free morpholino ring nitrogen to give a fluorophosphoramidate intersubunit linkage.

The starting material is one mmole of 5'hydroxyl subunit, base-protected with groups removable by a beta elimination mechanism and tritylated on the morpholino nitrogen (Structure 4 of Figure 5). The subunit is dissolved in 20 ml of dichloromethane to which is added 6 mmole of N-methylimidazole, followed by the addition of 2.5 mmole of fluorophosphoric acid. 5 mmole of DCC is added and the solution stirred three hours. The reaction solution is washed with aqueous NaH₂PO₄ and the organic phase dried under reduced pressure to give the fluorophosphate salt. The product is purified by silica gel chromatography developed with a methanol/chloroform mixture 1% in pyridine to give the pyridinium salt. After drying the purified product is suitable for coupling to a 5'-protected subunit, having a free morpholino nitrogen , using DCC in dichloromethane.

Polymers containing the fluorophosphoramidate intersubunit linkage should not be exposed to strong nucleophiles, such as ammonia. Consequently, bases of the subunits used for assembling such polymers should be protected with groups which can be cleaved without the use of strong nucleophiles. Protective groups cleavable via a beta elimination mechanism, as described in Example 1, are suitable for this purpose.

### D. X = N(CH₃)₂

Example 5D describes the coupling of a 5'hydroxyl subunit, prepared as in Example 2 or 3, to a second subunit having a free morpholino ring nitrogen to give a phosphordiamidate intersubunit linkage. The example is described with reference to structures in Figure 8, where X is a disubstituted nitrogen.

One mmole of 5'hydroxyl subunit, base-protected and tritylated on the morpholino nitrogen (Structure 4 of Figure 5) is dissolved in 5 ml of dichloromethane. Six mmole of N-ethylmorpholine and 2 mmole of dimethylaminodichlorophosphate (OP (Cl)₂N (CH₃)₂) for Z = O (or the thiophosphate analog for Z = S) is added to the solution, followed by the addition of 0.5 mmole of N-methylimidazole. After the reaction is complete (assessed by thin layer chromatography) the reaction solution is washed with aqueous NaH₂PO₄. The activated subunit is isolated by chromatography on silica gel developed with acetone/chloroform (Structure 2 in Figure 8, where X is N(CH₃)₂). The activated subunit is directly linked to the morpholino nitrogen of a subunit (Structure 3) in DMF containing triethylamine sufficient to neutralize the HCl produced in the reaction, to give the dimer shown as Structure 4.

The dimethylaminodichlorophosphate (X is -N(CH₃)₂ and Z is oxygen) used in the above procedure was prepared as follows: a suspension containing 0.1 mole of dimethylamine hydrochloride in 0.2 mole of phosphorous oxychloride was refluxed for 12 hours and then distilled (boiling point is 36°C at 0.5 mm Hg). The dimethylaminodichlorothiophosphate (X is -N(CH₃)₂ and Z is sulfur) used above was prepared as follows: a suspension containing 0.1 mole of dimethylamine hydrochloride in 0.2 mole of thiophosphoryl chloride was refluxed for 18 hours and then distilled (boiling point 85°C at 15 mm Hg).

An alternative activation and coupling procedure entails dissolving one mmole of 5'hydroxyl subunit, base-protected and tritylated on the morpholino nitrogen (Structure 4 of Figure 5), in 20 ml of dichloromethane. To this solution 4 mmole of N,N-diethylaniline and 1 mmole of 4-methoxypyridine-N-oxide are added. After dissolution, 2 mmole of dimethylaminodichlorophosphate, for Z = O (or dimethylaminodichlorothiophosphate, for Z = S), is added. After two hours the product (Structure 2 of Figure 8, where X is dimethylamine) is isolated by chromatography on silica gel developed with 10% acetone/90% chloroform. One mmole of this activated subunit can be directly linked to the morpholino nitrogen of a second subunit (Structure 3) by mixing them in dichloromethane containing 1.5 mmole of N,N-diisopropylaminoethyl isobutyrate (DIPAEIB) to give the dimer shown as Structure 4 (where X is -N-(CH₃)₂).

### Example 6

### Solution-Phase Assembly of a Simple Prototype Morpholino Polymer with Structural Confirmation, Deprotection, Purification, and Assessment of Its Binding to a Target RNA Sequence

This example describes the preparation, structural confirmation, and assessment of target binding affinity of a simple phosphordiamidate-linked morpholino polymer.

A morpholino hexamer where all Pi moieties are cytosines is assembled from dimer prepared as in Example 5D. One third of that dimer preparation is detritylated with trifluoroethanol (TFE) containing 2% acetic acid; the TFE is then removed under reduced pressure. The residue is washed with ether to remove any residual acetic acid. The remaining two thirds of the dimer preparation is activated (as in Example 5D). Half of the activated dimer is reacted with the detritylated dimer to give a tetramer product which is purified by silica gel chromatography developed with 6% methanol/94% chloroform. The tetramer is detritylated and reacted with the remaining activated dimer to give a hexamer product which is purified by silica gel chromatography developed with 10% methanol/90% chloroform.

This phosphordiamidate-linked 5'OH, base-protected hexamer having a trityl moiety on the morpholino nitrogen is designated pd(mC)₆-trityl. The negative ion Fast Atom Bombardment mass spectrum (3-nitrobenzyl alcohol matrix) shows: M-1 = 2667.9 (100).

This pd(mC)₆-trityl polymer is next detritylated as above, followed by base deprotection.

If it is desired to add a solubilizing group to the morpholino polymer this can be done conveniently as follows. The N-terminal morpholino nitrogen is detritylated using 2% acetic acid in trifluoroethanol. Polyethylene glycol 1000 (from Polysciences Inc., Warrington, Pennsylvania, USA) is thoroughly dried by dissolving in dry DMF and then evaporating the solvent under vacuum. The solid is resuspended in a minimal volume of pure dry DMF and 0.5 mole equivalent (relative to PEG 1000) of bis(p-nitrophenyl)carbonate and 1 mole equivalent of TEA are added. The preparation is sealed and incubated overnight at 30°C to give p-nitrophenyl carbonate-activated PEG 1000.

The full-length morpholino polymer which has been detritylated is added to a substantial molar excess (generally 10 to 20 fold) of activated PEG 1000 and incubated two hours at room temperature. The entire preparation is then treated with concentrated ammonium hydroxide overnight at 30°C. Ammonia is then removed under reduced pressure. Purification is achieved by cation exchange chromatography followed by desalting on a column of polypropylene washed with water and then eluted with methanol.

This purified tailed hexamer, pd(mC)₆-PEG1000, shows an absorption maximum at 267.1 nm in neutral aqueous solution, with a calculated molar absorbance of 42,800. In aqueous solution at pH 1, the same material shows an absorption maximum at 275.7 nm, with a calculated molar absorbance of 77,100.

To assess target-binding affinity of the pd(mC)₆ PEG-1000 polymer, 1 mg of polyG RNA (purchased from Sigma Chem Co.) is dissolved in deionized water, and 4 volumes of DMSO (spectrophotometric grade from Aldrich Chem. Co.) is added (stock solution A). The tailed morpholino hexamer, pd(mC)₆-PEG1000, is dissolved in spectrophotometric grade DMSO (stock solution B). Phosphate buffer is prepared by adjusting the pH of 0.05 N NaOH to 7.4 using phosphoric acid (Buffer C).

Stock solutions A and B are assayed for the actual concentration of polymer by UV; the absorbance of stock solution A is measured in 0.1 N NaOH and stock solution B is measured in 0.1 N HCl. Measurements at these pH extremes minimize base stacking and other polymer interactions which can give absorbance values not proportional to the component monomers. Stock solutions A and B are diluted with Buffer C to give solutions of a final concentration of 10 micromolar in polymer. The required dilutions are calculated using molar absorbancies of 65,000 for solution A, poly(G), and 77,100 for solution B, pd(mC)₆-PEG1000.

Assessment of target binding affinity is carried out in a double-beam scanning spectrophotometer having a temperature-controlled cell housing which will accommodate two cells in the reference beam and two in the sample beam.

Using four matched quartz cuvettes, one is filled with 0.5 ml of poly(G), 60 micromolar with respect to G monomer, and 0.5 ml of Buffer C and a second is filled with 0.5 ml of 10 micromolar pd(mC)₆-PEG1000 and 0.5 ml of Buffer C. These two cuvettes are placed in the reference beam of the temperature-controlled cell housing. Next, a third cuvette is filled with 1 ml of Buffer C and a fourth is filled with 0.5 ml of poly(G) 60 micromolar with respect to G monomer and 0.5 ml of 10 micromolar pd(mC)₆-PEG1000. These two cuvettes are placed in the sample beam of the cell housing. The cell housing is then heated to 50 °C and allowed to cool slowly to 14°C to assure complete pairing between the polymer and its target polynucleotide in the fourth cuvette. A scan is then taken from 320 nm to 240 nm-which shows a substantial absorbance difference due to a hypochromic shift in polymer-target mixture, centered around 273 nm. The temperature of the cell holder is then raised in 2-degree increments to 72°C, with scans taken after each 2 degree rise.

A plot of the absorbance difference as a function of temperature for the morpholino polymer/RNA complex is shown in Figure 9. The melting temperature, Tm, where the complex is half melted, is seen to be 51.5°C for this morpholino polymer/RNA.

### Example 7

### Solution-Phase Preparation of Phosphordiamidate-Linked Oligomer of the Sequence 5'-CGCCA

This example describes the assembly of a short oligomer containing a phosphordiamidate-linked backbone (Structure of Figure 4, where X is N(CH₃)₂, Y is oxygen and Z is oxygen). This solution assembly method is useful for large-scale synthesis of short oligomers suitable for subsequent assembly into longer oligomers, as in Examples 8 and 9.

### A. Preparation of the monomer for use at the 5' end of the block

A morpholino C subunit containing a trityl moiety on the morpholino ring nitrogen and having a methylamine on the 5' methylene (prepared as in Example 4) is reacted with N- (9-fluorenylmethoxycarbonyloxy) succinimide (FMOC) from Aldrich Chem. Co., Milwaukee, Wisconsin, USA., and purified by silica gel chromatography.

### B. Detritylation

To a separatory funnel add 1 mmole of the FMOC-derivatized C subunit, prepared in part A above, 18 ml of dichlormethane, 2 ml of trifluoroethanol, and 0.4 g of cyanoacetic acid. After 10 minutes this solutions is partitioned with aqueous NaHCO₃. The organic phase is removed by roto-evaporation. To the remaining material 20 ml acetonitrile is added and the acetonitrile removed by roto-evaporation to dryness. The remaining material is dried under vacuum for 1 hour.

### C. Activation of other monomers

5'OH morpholino subunits of A, C, and G, tritylated on the morpholino ring nitrogen, are prepared as in Example 2. The A, C, and G subunits are activated by conversion to the monochlorcphosphoramidate form, as in Example 9D.

### D. First coupling

The detritylated FMOC-derivatized C subunit from part B above is suspended in coupling solution (4 ml dichloromethane, 2 ml tetramethylene sulfone, and 0.3 ml DIPAEIB (prepared as in Example 5)). To this solution 1 mmole of activated G subunit, prepared in part C above, is added. After 30 minutes at room temperature the dichloromethane is removed under reduced pressure and the product is precipitated by adding water. The 5'FMOC-CG-Trityl dimer is purified by chromatography on silica gel developed with 5% methanol/95% chloroform.

### E. Subsequent couplings

The FMOC-CG-Trityl product is de-tritylated as in part B above and coupled with 1 mmole of activated C subunit from part C above. This coupling cycle is repeated to add the remaining C and A subunits, to give the desired block: 5'FMOC-CGCCA-Trityl. As the length of the polymer increases, the purification step requires an increasing concentration of methanol in the silica gel chromatographic purification of the growing block.

### F. Structural confirmation

The structure of this pentamer block is most easily confirmed by NMR and negative ion fast atom bombardment mass spectroscopy. As a rule the dominant species in the mass spectrum is the molecular ion.

### Example 8

### Solution-Phase Block Assembly of Morpholino Polymer Targeted Against the Primer Binding Site of HIV-I

This example describes the use of oligomer blocks, prepared as in Example 7, for solution-phase assembly of a morpholino polymer containing phosphordiamidate intersubunit linkages. The use of short oligomer blocks instead of monomers greatly simplifies separation of the final product from failure sequences. The morpholino polymer synthesized in this example has a base sequence complementary to the primer binding site of HIV-I (Myers et al.).

### A. Synthesis of short oligomer blocks

The following oligomers are synthesized as per Example 7: 5'FMOC-GUU-Trityl (block 1); 5'FMOC-CGGG-Trityl (block 2); 5'FMOC-CGCCA-Trityl (block 3); 5'FMOC-CUGC-Trityl (block 4).

### B. Cleavage of the FMOC moiety

1.1 Millimole of block 4 is suspended in 20 ml DMF and 0.4 ml of DBU (1,8-diazabicyclo[5.4.0]undec-7-ene) is added. After 20 minutes 200 mls of ether is added, the solution stirred well, and filtered. The filtrate is resuspended in 20 ml of dichloromethane and distribute into four 30 ml centrifuge tubes, to each of which is added 25 mls of ether, shaken well to mix, and centrifuged. The supernatants are discarded and the pellets dried under reduced pressure.

### C. Detritylation and activation

Next, 1 mmole of block 3 as per Example 7, part B, is de-tritylated. After removal of the solvent under reduced pressure, the remaining material is suspended in 20 ml of dichloromethane, followed by the addition of 1.5 ml of DIPAEIB (prepared as in Example 5). While rapidly stirring, 0.5 ml of ethyldichlorophosphate is added. After 5 minutes, this solution is distributed into 4 - 30 ml centrifuge tubes. Each tube is filled with ether, shaken thoroughly, and centrifuged. The pellets are resuspended in a minimal volume of dichloromethane, followed by the addition of ether. The tubes are then shaken and centrifuged. This washing procedure is repeated once more. The pellet is suspended in coupling solution (10 ml dichloromethane, 5 ml tetramethylene sulfone, and 0.3 ml DIPAEIB (prepared as in Example 5)).

### D. Coupling

The solution of activated block 3 is added to block 4 from which the FMOC group has been cleaved. After one hour 0.5 ml of acetic anhydride is added and the solution let stand at room temperature for 5 minutes. The dichloromethane is removed under reduced pressure. To precipitate the product, 5'FMOC-CGCCACUGC-Trityl, water is added. After drying the solid under vacuum, to thoroughly remove all residual acetic anhydride, it is resuspended in dichloromethane. Ether is then added to precipitate the product. The product is resuspended once more in dichloromethane and re-precipitate with ether.

The FMOC moiety is removed from this 9-mer product as in part B above. Block 2 is detritylated and activated as in part C above. These two components are coupled as described above to give the 13-mer: 5'FMOC-CGGGCGCCACUGC-Trityl.

The FMOC moiety is cleaved from this 13-mer product as in part B above. Block 1 is detritylated and activated as in part C above. These two components are coupled as described above to give the 16-mer: 5'FMOC-GUUCGGGCGCCACUGC-Trityl.

### E. Deprotection

The FMOC and trityl moieties are removed as above and the product is suspended in 20 ml DMF and 20 ml of ammonium hydroxide are added. The preparation is capped and incubated at 30°C for 18 hrs. Thereafter, the solvent is removed by roto-evaporation to give a crude preparation of the 16-mer: 5'-GUUCGGGCGCCACUGC.

### F. Purification

The 16-mer preparation is suspended at a concentration of 2 mg/ml in 0.05 N aqueous methylamine adjusted to pH 10.5 with HCl (Solvent A). Water to be used for chromatography is degassed under aspirator vacuum and methylamine is added to give a final methylamine concentration of 0.05 N. To this solution HCl is added to adjust the pH to 10.5. A corresponding pH 10.5 solution is made 1 N in KCl (solvent B). Solvent A is mixed 1:1, by volume, with chromatographic-grade acetonitrile or methanol to give solvent C.

Up to 100 mg of the polymer is loaded in 50 ml of Solvent A on a chromatography column (5 cm in diameter and 15 cm in length) packed with the anion-exchange support Q-Sepharose Fast Flow (Pharmacia). The column is washed thoroughly with solvent A, and eluted with a linear gradient ranging from 100% solvent A to 100% solvent B over 30 minutes using a flow rate of about 30 ml/minute. The eluant is monitered at 254 nm. The 16-mer of this example was found to elute at about 0.35 N KCl. The desired binding polymer is generally the last and the largest peak to elute from the column. When the polymer is prepared by block assembly, baseline separations are often achieved. Generally when peak shapes are unsymmetrical the problem is the insolubility of the binding polymer rather than a lack of capacity of the chromatographic packing. Such a problem can often be solved by reducing the quantity of binding polymer loaded in a given run. When peaks are symmetrical but base-line separation is not achieved, improvement can usually be obtained by eluting using a more shallow gradient.

The eluant containing the polymer is further purified and desalted by loading on an equivalent-sized column packed with 35 micron chromatographic grade polypropylene (cat. no. 4342 from Polysciences, Inc.). The column is washed thoroughly with solvent A. If baseline separation was achieved in the foregoing cation exchange chromatography, then pure product is obtained by eluting with solvent C. If, however, baseline separation was not achieved, the product is eluted with a linear gradient ranging from 100% solvent A to 100% solvent C. The 16-mer in this example was found to elute when the concentration of acetonitrile was about 25% by volume.

### G. Sequence confirmation

Mass spectral analysis of the full-length polymer in the fully-protected state, as described earlier, can serve to confirm both the polymer length and the base composition, but it provides no information about the subunit sequence. One method to obtain sequence information is from the fragmentation patterns of deoxyribonucleic acids and carbamate-linked deoxyribonucleoside-derived polymers (Griffin et al.). However, in the fully-protected state many morpholino-based polymers of the present invention are resistant to fragmentation and yield predominantly the molecular ion with only minimal fragments.

An alternative method to confirming the sequence of a morpholino-based polymer is to take a small portion of the growing polymer after coupling each oligomer block and use mass spectral analysis to follow the elongation of the polymer. This method is applicable except for rare cases where two blocks used in the synthesis happen to have exactly the same mass.

A preferred, but indirect, method to help confirm the correctness of the polymer subunit sequence is to pair the morpholino polymer with its complementary DNA (whose sequence can be confirmed by established methods) and with DNA sequences which might have resulted if the blocks were assembled in the wrong order. Pairing between the polymer and DNA can be evaluated by assessing for a hypochromic shift in the 240 to 290 nm wavelength region (assessed as in Example 6). Such a shift occurs only between the polymer and its complementary sequence. The polymer/DNA duplex can also be. distinguished from any partially-mismatched duplex by slowly raising the temperature while monitoring the absorbance in the 240 nm to 290 run region. The perfect duplex will have a melting temperature (corresponding to a 50% reduction in the hypochromicity) generally 10 degrees or more above that of any mismatched duplex.

### H. Assessment of target binding affinity

The 16-mer of this Example, when paired with its complementary DNA was found to bind DNA when the strands are antiparallel, but not when they are parallel (as illustrated below).

Binding: morpholino polymer: 5′-GUUCGGCGCCACUGC
target DNA: 3′-CAAGCCGCGGTGACG-5′

No binding:    morpholino polymer: 5'-GUUCGGGCGCCACUGC
                        target DNA: 5'-CAAGCCCGCGGTGACG-3'

The anti-parallel bound strand molecule was found have a Tm (determined as described in Example 6) of 63°C when each strand was present at a concentration of 3 micromolar.

### I. Assessment of biological activity

The 16-mer of this example was tested for its ability to inhibit synthesis of the viral P24 protein in human cells infected with HIV-I -- the AIDS virus. Preliminary results indicate that when the 16-mer polymer of this example, which is complementary to the primer binding site of HIV-I, is added at a concentration of 6 micromolar to HIV-I-infected cells the polymer causes a 35% reduction in synthesis of viral P24 protein. Further, the polymer, at concentrations of 6 and 19 micromolar, causes no detectable cell toxicity in uninfected cells.

### Example 9

### Solid-Phase Block Assembly of Morpholino Polymer Targeted Against the Primer Binding Site of HIV-I

This example describes the use of oligomer blocks, prepared as in Example 7, for solid-phase assembly of a morpholino polymer containing phosphordiamidate intersubunit linkages. Solid-phase assembly provides a rapid method for assembly of longer binding polymers. The use of short oligomer blocks instead of monomers greatly simplifies separation of the final product from failure sequences.

### A. Attachment of cleavable linker to solid support

One mmole of Bis [2-(succinimidooxycarbonyloxy)-ethyl]sulfone (Pierce Chemical Co. of Rockford, Illinois, USA) is dissolved in 10 ml of DMF and added to 3 g of a suitable solid support containing primary amine functions: e.g., Glass, Aminopropyl, Catalog # G4643, from Sigma Chem. Co., St. Louis, MO, USA -- 500 Angstrom pore size, 200-400 mesh, amine content of 81 micromole/g. After 2 hours the slurry of glass beads is poured into a column (preferably about 1.5 cm in diameter), the column washed thoroughly with dichloromethane, and dried under vacuum. This linker bound to the glass support is stable to the acidic conditions used for detritylations, but can be readily cleaved via a beta elimination mechanism using a strong non-nucleophilic base, such as a 1,8-diazabicyclo[5.4.0]undec-7-ene (DBU).

### B. Synthesis of short oligomer blocks

The following oligomers are synthesized as per Example 7: 5'FMOC-GUU-Trityl (block 1); 5'FMOC-CGGG-Trityl (block 2); 5'FMOC-CGCCA-Trityl (block 3); 5'FMOC-CUGC-Trityl (block 4).

### C. Addition of the first block to the support-bound linker

One millimole of block 1 oligomer is suspended in 20 ml DMF and 0.4 ml of DBU (1,8-diazabicyclo[5.4.0]undec-7-ene) is added. After 5 minutes 200 ml of ether is added, the suspension stirred well, and filtered. The filtrate is resuspended in 20 ml of dichloromethane and distributed into four 30 ml centrifuge tubes. To each tube 25 ml of ether is added, shaken well, and centrifuged. The supernatants are discarded and the pellets dried under reduced pressure. This oligomer is resuspended in 12 ml of DMF and added to the column of solid synthesis support prepared in part A above. The bed of glass beads is agitated periodically to assure thorough access of the oligomer block to all internal surfaces of the beads. After 2 hours at room temperature wash the column thoroughly with dichloromethane. The unbound oligomer block, present in the wash, can be recovered and reused.

### D. Detritylation and activation

The support-bound oligomer block is detritylated by slowly passing a solution through the column which consists of 88 ml of dichloromethane, 10 ml of trifluoroethanol, and 2 g of cyanoacetic acid. The column is washed with 40 ml of dichloromethane, followed by 40 ml of 1% N-ethylmorpholine in dichloromethane. The column is then washed with 50 ml of dichloromethane. The terminus of the bound oligomer is activated by slowly passing 100 ml of dichloromethane containing 5 ml of DIPAEIB (prepared as in Example 5) and 3 ml of ethyldichlorophosphate through the column. The column is then washed with 100 ml of chloroform.

### E. Addition of subsequent blocks to support-bound oligomer

One millimole of oligomer block 2 is suspended in 20 ml DMF and 0.4 ml of DBU (1,8-diazabicyclo[5.4.0]undec-7-ene) is added to the suspension. After 5 minutes 200 mls of ether is added, the suspension stirred well, and filtered. The filtrate is resuspended in 20 ml of dichloromethane and distributed into four 30 ml centrifuge tubes. To each tube 25 ml ether is added. The tubes are shaken well and centrifuged. The supernatants are discarded and the pellets dried under reduced pressure. The oligomer is resuspended in coupling solution (8 ml of dichloromethane, 4 ml of tetramethylene sulfone, and 0.5ml of DIPAEIB (prepared as in Example 5)). This solution is slowly introduced into the bed of the synthesis column until only 1 to 2 ml of volume remains above the column bed. The support is agitated periodically to assure complete access of the coupling solution to all internal pore surfaces of the beads. After 2 hours at room temperature, the column is washed choroughly with dichloromethane.

Blocks 3 and 4 are added in the same manner as described above.

### F. Cleavage from the support

A solution for cleaving the linker (by volume: 6 parts diethylmalonate; 12 parts DBU; 41 parts tetramethylene sulfone; and, 41 parts dichloromethane) is slowly passed through the column while monitoring the eluant at 290 nm via a 1 mm path-length flow cell. The eluant is collected until all of the polymer is eluted (generally about 30 minutes). The dichloromethane is removed under reduced pressure. To the remaining material aqueous NaH₂PO₄ sufficient to neutralize the DBU is added and then a large volume of water is added to precipitate the polymer. The solid product is collected and dried.

### G. Structural characterization, deprotection, purification, and testing

The fully-protected 16-mer product is deprotected, purified, and analyzed as described in Example 8. After deprotection and purification, the 16-mer polymer of this example shows essentially the same Tm with its target DNA and essentially the same biological activity as the 16-mer polymer prepared as described in Example 8.

### Example 10

### Stepwise Solid-Phase Synthesis of Morpholino Polymer Targeted Against a Splice Junction in Herpes simplex I

This example describes the stepwise assembly on a solid support of a polymer containing a phosphordiamidate-linked backbone (Structure of Figure 4, where X is N(CH₃)₂, Y is oxygen and Z is oxygen). This polymer having the sequence, 5'-CGUUCCUCCUGC, is targeted against a splice junction site in the virus, Herpes simplex I (Atencio et al.).

### A. Attachment of cleavable linker to solid support

A cleavable linker is attached to the solid support as described in Example 9, part A.

### B. Coupling the first monomer to the support.

One mmole of a morpholino C subunit containing a trityl moiety on the morpholino ring nitrogen and having a methylamine on the 5' methylene (prepared as in Example 4) is suspended in 12 ml of DMF and added to a column prepared for solid synthesis support as described in Example 9, part A). The bed of glass beds is agitated periodically to assure thorough access of the first subunit to all internal surfaces of the beads. After 1 hour at room temperature the column is washed thoroughly with dichloromethane. Unbound subunits, which may be present in the wash, can be recovered and reused.

### C. Coupling Cycle

### a. Detritylation

The support-bound subunit is detritylated by slowly passing a solution consisting of 88 ml of dichloromethane, 10 ml of triflucroethanol, and 2 g of cyanoacetic acid through the column. The column is washed with 40 ml of dichloromethane, followed by 40 ml of 1% N-ethylmorpholine in dichloromethane, and finally, with 100 ml of chloroform.

### b. Addition of subunit to the growing chain

One mmole of subunit (prepared as in Example 2 and activated with N,N-dimethylaminodichlorophosphate as described in Example 5, part D) is suspended in coupling solution (8 ml of dichloromethane, 4 ml of tetramethylene sulfone, and 0.5 ml of DIPAEIB (prepared as in Example 5)). This solution is slowly introduced into the bed of the synthesis column until only 1 to 2 ml of volume remains above the bed. The support is agitated periodically to assure complete access of the coupling solution to all internal pore surfaces of the beads. After 1 hour at room temperature, the column is washed thoroughly with dichloromethane. Any unbound subunit in this wash can be recovered and reused.

### c. Capping of unreacted chain termini

Fifty mililiters of dichloromethane containing 2 ml acetic anhydride and 2 ml of DIPAEIB (prepared as in Example 5) is added to the column. The column is washed with 100 ml of methanol containing 2 ml of DIPAEIB, followed by a wash using 200 ml of dichloromethane,

Using this coupling cycle (comprising steps a, b, and C above), the subunits G, U, U, C, C, U, C, C, U, G, and C, are added in sequence.

### D. Cleavage from the support

The completed polymer is cleaved from the support as described in Example 9.

### E. Structural characterization, deprotection, purification, and testing

The fully-protected 12-mer product is deprotected, purified, and analyzed as described in Example 8. After deprotection and purification, the Herpes-targeted 12-mer polymer, prepared as above, was found have a Tm (determined as described in Example 6) of 47°C when paired with its complementary DNA (the two strands anti-parallel) : each strand was present at a concentration of 2 micromolar.

### F. Preliminary assessment of biological activity

The 12-mer of this example was tested for its ability to protect mice infected by the target virus, Herpes SimpleX Virus I (HSVI). Mice infected in the eye with HSV I showed a survival of 65% at 5 days post infection. When Herpes-infected mice were treated with a topical ointment containing 0.3 mmolar of the 12-mer described in this example, the survival was increased to 90% at 5 days post infection.

## Claims (Claims for the following Contracting State(s): AT, BE, CH & LI, DE, DK, FR, GB, IT, LU, NL & SE)

1. A polymer comprised of moipholino subunit structures of the form: 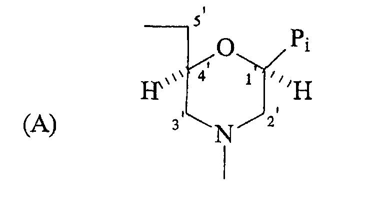 where (i) the structures are linked together by uncharged, chiral linkages joining the morpholino nitrogen of one subunit to the 5'-exocyclic carbon of an adjacent subunit, and (ii) Pᵢ is a purine or pyrimidine base-pairing moiety effective to bind by base-specific hydrogen bonding to a base in a polynucleotide, and wherein the linkage (i) is 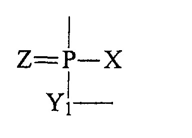 wherein X is F, CH₂R, OCH₂R, -S-CH₂R, NR₁R₂, morpholino, pyrrolyl or pyrazolyl; where R, R₁ and R₂ may be the same or different and are selected from H and CH₃; Y₁ is joined to the 5'-exocyclic carbon of the morpholino subunit; Y₁ is O, S, CH₂ or NR; and Z is O or S.

2. The polymer of claim 1, wherein Pᵢ is selected from: 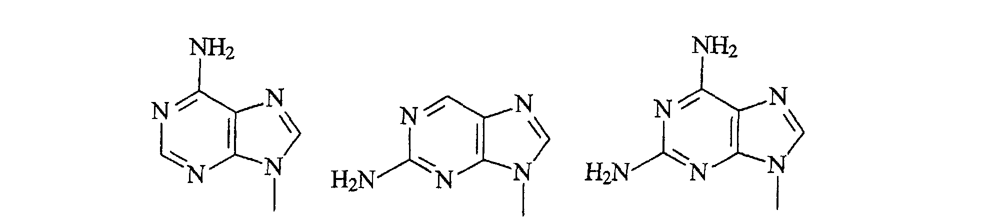 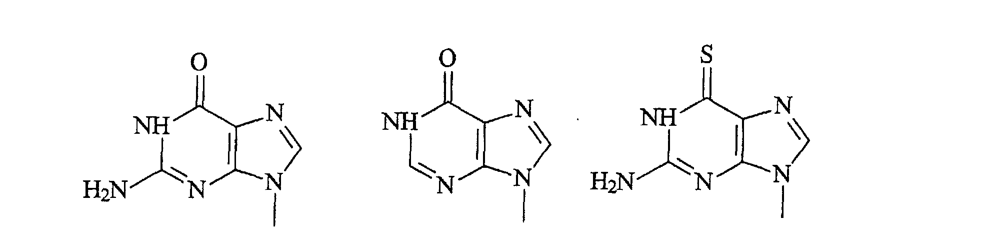 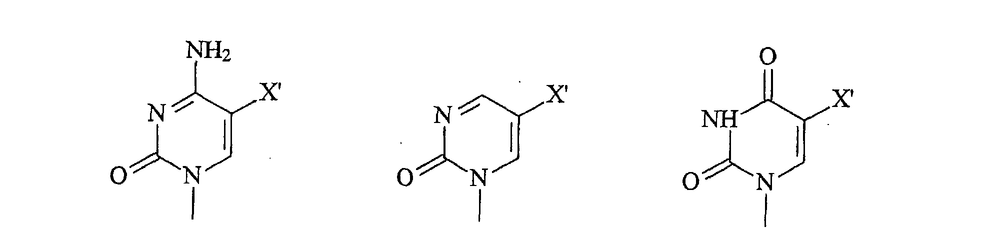 where X' is H, CH₃, F, Cl, Br or I.

3. The polymer of claim 1 or 2, where Z is O and Y₁ is O.

4. The polymer of claim 1 or 2, wherein the linkage is of the form: 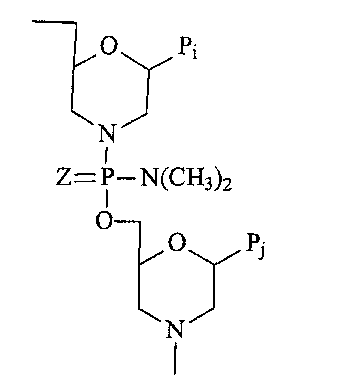 wherein Pⱼ is a purine or pyrimidine base-pairing moiety effective to bind by base specific hydrogen bonding to a base in a polynucleotide.

5. The polymer of claim 1 or 2, wherein the linkage is of the form: 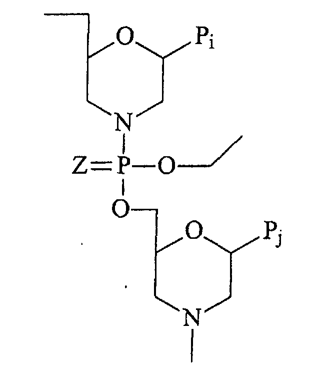 wherein Pⱼ is a purine or pyrimidine base-pairing moiety effective to bind by base specific hydrogen bonding to a base in a polynucleotide.

6. The polymer of any one of the preceding claims, which further includes a moiety at one or both termini which is effective to enhance the solubility of the said polymer in aqueous medium.

7. The polymer of claim 6, wherein the moiety at one or both teimini is polyethylene glycol.

8. The polymer of any one of the preceding claims, composed of at least 3 morpholino subunits.

9. The polymer of any one of the preceding claims, wherein at least one of the Pᵢ moieties is a 2,6-diaminopurine moiety.

10. The polymer of any one of the preceding claims, wherein at least one of the Pᵢ moieties is a 5-halouracil moiety.

11. The polymer of any one of the preceding claims, wherein at least 70% of the Pᵢ moieties are 2-amine containing purine moieties.

12. The polymer of any one of the preceding claims attached to a solid support.

13. A method for detecting, in a sample, the presence of a polynucleotide having a selected target sequence, comprising contacting a polymer of any one of claims 1 to 12 with said polynucleotide, where said polymer (i) has a series of base-pairing moieties capable of binding to the selected target sequence, and (ii) is labeled with a detectable reporter group, and where said reacting of the polymer with the polynucleotide is carried out under conditions effective to allow formation of a hybridization complex between the polymer and the target sequence, and detecting the presence of the reporter group.

14. The method of claim 13, wherein said polynucleotide is single-stranded.

15. The method of claim 14, wherein said polynucleotide is DNA.

16. The method of claim 14, wherein said polynucleotide is RNA.

17. The method of claim 13, wherein said polynucleotide is double-stranded.

18. The method of claim 13, wherein said polynucleotide is bound to a solid support.

19. A method of isolating, from a sample, a nucleic acid having a selected target sequence, comprising
contacting a polymer of any one of claims 1-11 with said nucleic acid, where said polymer (i) has a series of base-pairing moieties capable of binding to the selected target sequence, and (ii) is attached to a solid support, and (iii) where said contacting of the polymer with the nucleic acid is carried out under conditions effective to allow formation of a hybridization complex between the polymer and the target sequence, and
isolating said nucleic acid.

## Claims (Claims for the following Contracting State(s): ES & Gr)

1. A polymer comprised of morpholino subunit structures of the form: 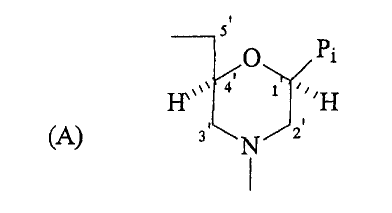 where (i) the structures are linked together by uncharged, chiral linkages joining the morpholino nitrogen of one subunit to the 5'-exocyclic carbon of an adjacent subunit, and (ii) Pᵢ is a purine or pyrimidine base-pairing moiety effective to bind by base-specific hydrogen bonding to a base in a polynucleotide, and wherein the linkage (i) is 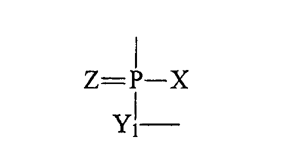 wherein X is F, CH₂R, OCH₂R, -S-CH₂R, NR₁R₂, morpholino, pyrrolyl or pyrazolyl; where R, R₁ and R₂ may be the same or different and are selected from H and CH₃; Y₁ is joined to the 5'-exocyclic carbon of the morpholino subunit; Y₁ is O, S, CH₂ or NR; and Z is O or S.

2. The polymer of claim 1, wherein Pᵢ is selected from: 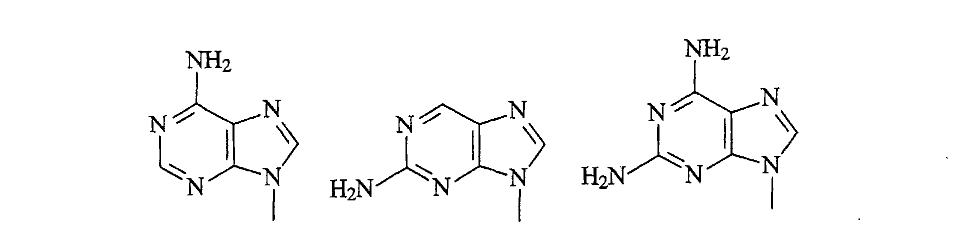 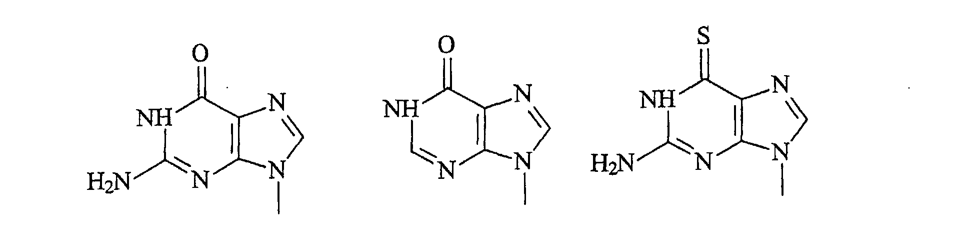 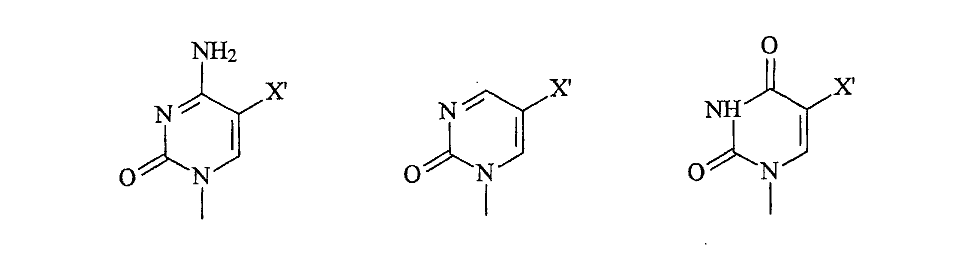 where X' is H, CH₃, F, Cl, Br or I.

3. The polymer of claim 1 or 2, where Z is O and Y₁ is O.

4. The polymer of claim 1 or 2, wherein the linkage is of the form: 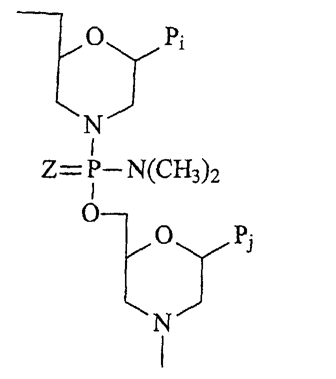 wherein Pⱼ is a purine or pyrimidine base-pairing moiety effective to bind by base specific hydrogen bonding to a base in a polynucleotide.

5. The polymer of claim 1 or 2, wherein the linkage is of the form: 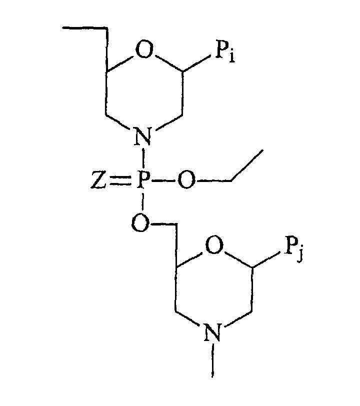 wherein Pⱼ is a purine or pyrimidine base-pairing moiety effective to bind by base specific hydrogen bonding to a base in a polynucleotide.

6. The polymer of any one of the preceding claims, which further includes a moiety at one or both termini which is effective to enhance the solubility of the said polymer in aqueous medium.

7. The polymer of claim 6, wherein the moiety at one or both termini is polyethylene glycol.

8. The polymer of any one of the preceding claims, composed of at least 3 morpholino subunits.

9. The polymer of any one of the preceding claims, wherein at least one of the Pᵢ moieties is a 2,6-diaminopurine moiety.

10. The polymer of any one of the preceding claims, wherein at least one of the Pᵢ moieties is a 5-halouracil moiety.

11. The polymer of any one of the preceding claims, wherein at least 70% of the Pᵢ moieties are 2-amine containing purine moieties.

12. The polymer of any one of the preceding claims attached to a solid support.

13. A process for preparing a polymer according to any one of claims 1 to 11, which process comprises linking together morpholino subunits structures of the form: 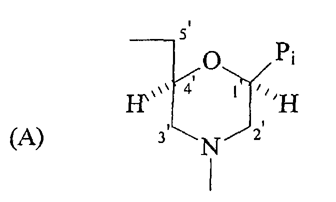 wherein Pᵢ is as defined in any one of claims 1 to 11, via uncharged chiral linkages of the formula 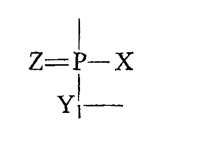 wherein Z, X and Y₁ are as defined in any one of claims 1 to 11 and Y₁ is joined to the 5'-exocyclic carbon of the morpholino subunit,
which linkages join the morphino nitrogen of one subunit to the 5'-exocyclic carbon of an adjacent subunit.

14. A method for detecting, in a sample, the presence of a polynucleotide having a selected target sequence, comprising contacting a polymer of any one of claims 1 to 12 with said polynucleotide, where said polymer (i) has a series of base-pairing moieties capable of binding to the selected target sequence, and (ii) is labeled with a detectable reporter group, and where said reacting of the polymer with the polynucleotide is carried out under conditions effective to allow formation of a hybridization complex between the polymer and the target sequence, and
detecting the presence of the reporter group.

15. The method of claim 14, wherein said polynucleotide is single-stranded.

16. The method of claim 15, wherein said polynucleotide is DNA.

17. The method of claim 15, wherein said polynucleotide is RNA.

18. The method of claim 14, wherein said polynucleotide is double-stranded.

19. The method of claim 14, wherein said polynucleotide is bound to a solid support.

20. A method of isolating, from a sample, a nucleic acid having a selected target sequence, comprising
contacting a polymer of any one of claims 1-11 with said nucleic acid, where said polymer (i) has a series of base-pairing moieties capable of binding to the selected target sequence, and (ii) is attached to a solid support, and (iii) where said contacting of the polymer with the nucleic acid is carried out under conditions effective to allow formation of a hybridization complex between the polymer and the target sequence, and
isolating said nucleic acid.

## Patentansprüche (Patentansprüche für folgende(n) Vertragsstaat(en): AT, BE, CH & LI, DE, DK, FR, GB, IT, LU, NL, & SE)

1. Polymer, umfassend Morpholino-Untereinheitsstrukturen der Form: 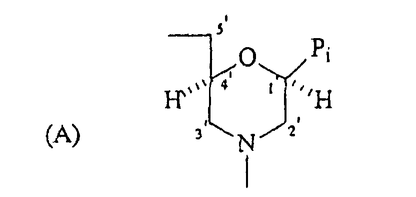 worin (i) die Strukturen miteinander verbunden sind durch ungeladene, chirale Verknüpfungen, welche den Morpholinostickstoff einer Untereinheit an den 5'-exozyklischen Kohlenstoff einer angrenzenden Untereinheit binden, und (ii) Pᵢ ein Purin- oder Pyrimidinbasen-Paarungsrest ist, welcher wirksam ist, durch basenspezifische Wasserstoffbindung an einer Base in einem Polynukleotid zu binden, und worin die Verknüpfung (i) 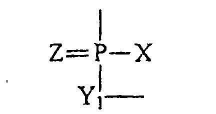 ist, worin X F, CH₂R, OCH₂R, -S-CH₂R, NR₁R₂, Morpholino, Pyrrolyl oder Pyrazolyl ist; worin R, R₁ und R₂ gleich oder unterschiedlich sein können und gewählt sind aus H und CH₃; Y₁ an den 5'-exozyklischen Kohlenstoff der Morpholino-Untereinheit gebunden ist; Y, O, S, CH₂ oder NR ist; und Z O oder S ist.

2. Polymer gemäß Anspruch 1, in dem Pᵢ gewählt ist aus: 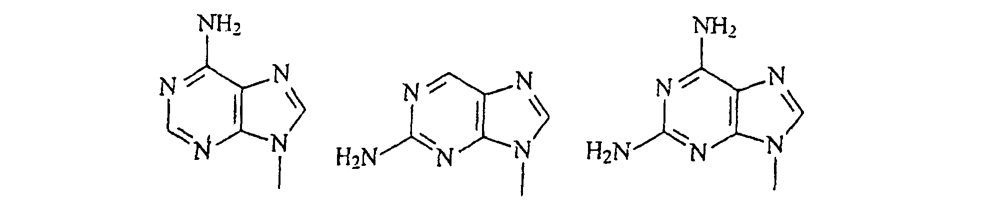 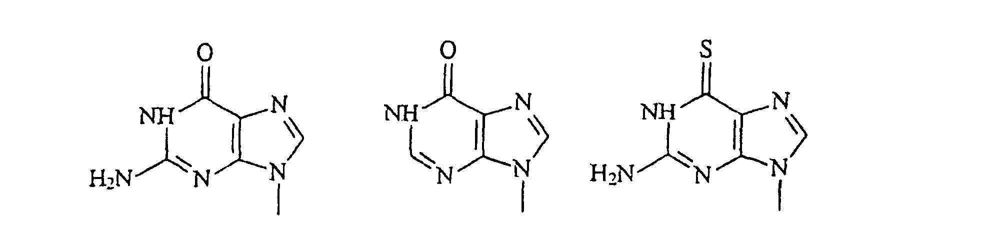 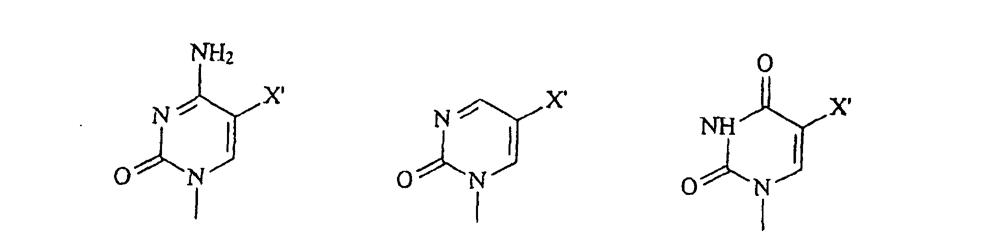 worin X' H, CH₃, F, Cl, Br oder I ist.

3. Polymer gemäß Anspruch 1 oder 2, in dem Z O ist und Y₁ O ist.

4. Polymer gemäß Anspruch 1 oder 2, in dem die Verknüpfung von folgender Form ist: 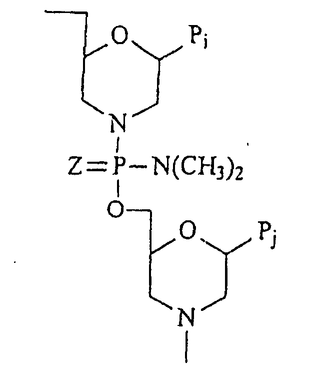 worin Pⱼ ein Purin- oder Pyrimidinbasen-Paarungsrest ist, der wirksam ist, durch basenspezifische Wasserstoffbindung an eine Base in einem Polynukleotid zu binden.

5. Polymer gemäß Anspruch 1 oder 2, in dem die Verknüpfung von folgender Form ist: 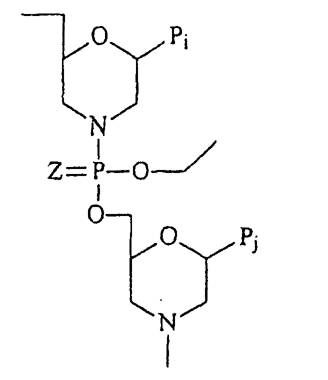 worin Pⱼ ein Purin- oder Pyrimidinbasen-Paarungsrest ist, der wirksam ist, durch basenspezifische Wasserstoffbindung an eine Base in einem Polynukleotid zu binden.

6. Polymer gemäß mindestens einem der vorstehenden Ansprüche, welches ferner einen Rest an einem oder an beiden Enden einschließt, welcher wirksam ist, die Löslichkeit des Polymers in wässrigem Medium zu erhöhen.

7. Polymer gemäß Anspruch 6, in dem der Rest an einem oder an beiden Enden Polyethylenglykol ist.

8. Polymer gemäß mindestens einem der vorstehenden Ansprüche, bestehend aus mindestens 3 Morpholino-Untereinheiten.

9. Polymer gemäß mindestens einem der vorstehenden Ansprüche, in dem mindestens einer der Pᵢ-Reste ein 2,6-Diaminopurin-Rest ist.

10. Polymer gemäß mindestens einem der vorstehenden Ansprüche, in dem mindestens einer der Pᵢ-Reste ein 5-Halogenuracil-Rest ist.

11. Polymer gemäß mindestens einem der vorstehenden Ansprüche, in dem mindestens 70% der Pᵢ-Reste 2-Amin enthaltende Purin-Reste sind.

12. Polymer gemäß mindestens einem der vorstehenden Ansprüche, das an einem festen Träger gebunden ist.

13. Verfahren zum Nachweisen des Vorliegens eines Polynukleotids mit einer gewählten Zielsequenz in einer Probe, umfassend das Kontaktieren eines Polymeren eines beliebigen der Ansprüche 1 bis 12 mit dem Polynukleotid, wobei das Polymer (i) eine Reihe von Basen-paarenden Resten aufweist, die zur Bindung an die gewählte Zielsequenz in der Lage sind, und (ii) mit einer nachweisbaren Reportergruppe markiert sind, und wobei das Umsetzen des Polymeren mit dem Polynukleotid unter Bedingungen durchgeführt wird, die wirksam sind, um die Bildung eines Hybridisierungskomplexes zwischen dem Polymer und der Zielsequenz zu ermöglichen, und
Nachweisen der Anwesenheit der Reportergruppe.

14. Verfahren gemäß Anspruch 13, bei dem das Polynukleotid einzelsträngig ist.

15. Verfahren gemäß Anspruch 14, bei dem das Polynukleotid DNA ist.

16. Verfahren gemäß Anspruch 14, bei dem das Polynukleotid RNA ist.

17. Verfahren gemäß Anspruch 13, bei dem das Polynukleotid doppelsträngig ist.

18. Verfahren gemäß Anspruch 13, bei dem das Polynukleotid an einen festen Träger gebunden ist.

19. Verfahren zur Isolierung einer Nukleinsäure mit einer gewählten Zielsequenz aus einer Probe, umfassend
das Kontaktieren eines Polymeren von einem beliebigen der Ansprüche 1-11 mit der Nukleinsäure, wobei das Polymer (i) eine Reihe von Basen-paarenden Resten besitzt, die zur Bindung der gewählten Zielsequenz in der Lage sind, und (ii) an einen festen Träger gebunden ist, und (iii), wobei das Kontaktieren des Polymeren mit der Nukleinsäure unter Bedingungen durchgeführt wird, die wirksam sind, um die Bildung eines Hybridisierungskomplexes zwischen dem Polymer und der Zielsequenz zu ermöglichen, und
Isolieren der Nukleinsäure.

## Patentansprüche (Patentansprüche für folgende(n) Vertragsstaat(en): ES & GR)

1. Polymer, umfassend Morpholino-Untereinheitsstrukturen der Form: 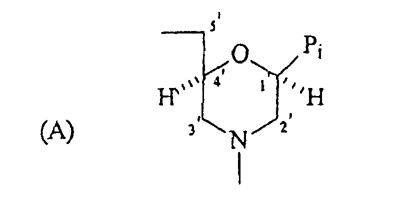 worin (i) die Strukturen miteinander verbunden sind durch ungeladene, chirale Verknüpfungen, welche den Morpholinostickstoff einer Untereinheit an den 5'-exozyklischen Kohlenstoff einer angrenzenden Untereinheit binden, und (ii) Pᵢ ein Purin- oder Pyrimidinbasen-Paarungsrest ist, welcher wirksam ist, durch basenspezifische Wasserstoffbindung an einer Base in einem Polynukleotid zu binden, und worin die Verknüpfung (i) 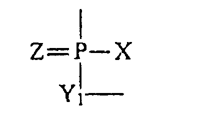 ist, worin X F, CH₂R, OCH₂R, -S-CH₂R, NR₁R₂, Morpholino, Pyrrolyl oder Pyrazolyl ist; worin R, R₁ und R₂ gleich oder unterschiedlich sein können und gewählt sind aus H und CH₃; Y₁ an den 5'-exozyklischen Kohlenstoff der Morpholino-Untereinheit gebunden ist; Y₁ O, S, CH₂ oder NR ist; und Z O oder S ist.

2. Polymer gemäß Anspruch 1, in dem Pᵢ gewählt ist aus: 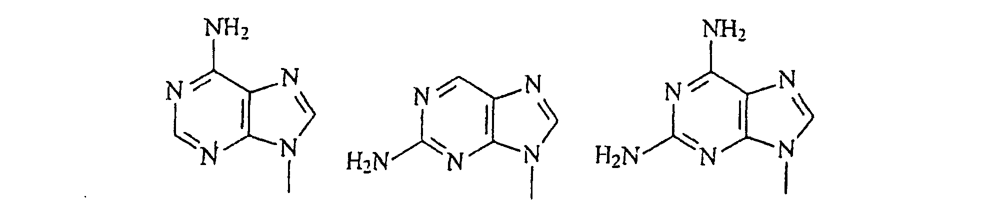 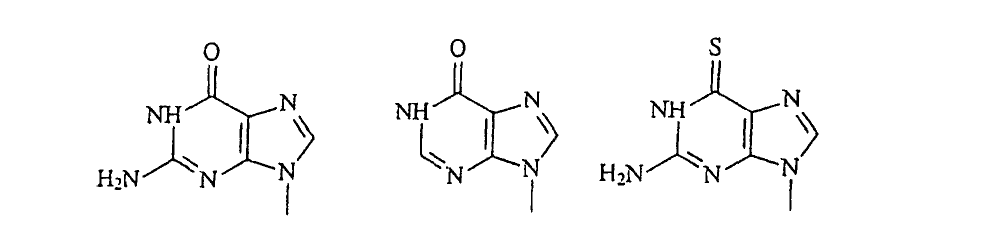 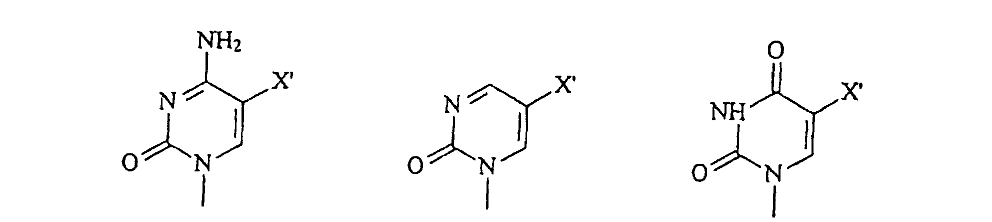 worin X' H, CH₃, F, Cl, Br oder I ist.

3. Polymer gemäß Anspruch 1 oder 2, in dem Z O ist und Y₁ O ist.

4. Polymer gemäß Anspruch 1 oder 2, in dem die Verknüpfung von folgender Form ist: 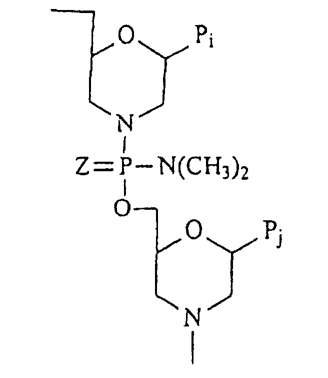 worin Pⱼ ein Purin- oder Pyrimidinbasen-Paarungsrest ist, der wirksam ist, durch basenspezifische Wasserstoffbindung an eine Base in einem Polynukleotid zu binden.

5. Polymer gemäß Anspruch 1 oder 2, in dem die Verknüpfung von folgender Form ist: 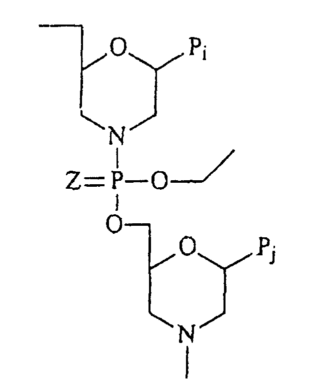 worin Pⱼ ein Purin- oder Pyrimidinbasen-Paarungsrest ist, der wirksam ist, durch basenspezifische Wasserstoffbindung an eine Base in einem Polynukleotid zu binden.

6. Polymer gemäß mindestens einem der vorstehenden Ansprüche, welches ferner einen Rest an einem oder an beiden Enden einschließt, welcher wirksam ist, die Löslichkeit des Polymers in wässrigem Medium zu erhöhen.

7. Polymer gemäß Anspruch 6, in dem der Rest an einem oder an beiden Enden Polyethylenglykol ist.

8. Polymer gemäß mindestens einem der vorstehenden Ansprüche, bestehend aus mindestens 3 Morpholino-Untereinheiten.

9. Polymer gemäß mindestens einem der vorstehenden Ansprüche, in dem mindestens einer der Pᵢ-Reste ein 2,6-Diaminopurin-Rest ist.

10. Polymer gemäß mindestens einem der vorstehenden Ansprüche, in dem mindestens einer der Pᵢ-Reste ein 5-Halogenuracil-Rest ist.

11. Polymer gemäß mindestens einem der vorstehenden Ansprüche, worin mindestens 70% der Pᵢ-Reste 2-Amin enthaltende Purin-Reste sind.

12. Polymer gemäß mindestens einem der vorstehenden Ansprüche, das an einem festen Träger gebunden ist.

13. Verfahren zur Herstellung eines Polymeren gemäß mindestens einem der Ansprüche 1 bis 11, wobei das Verfahren das Miteinanderverknüpfen von Morpholino-Untereinheitsstrukturen der Form: 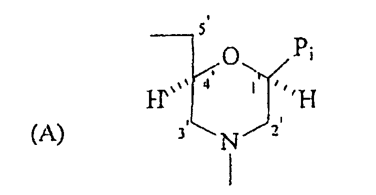 worin Pᵢ wie in einem beliebigen der Ansprüche 1 bis 11 definiert ist, vermittels ungeladener, chiraler Verknüpfungen der Formel 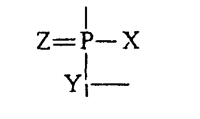 worin Z, X und Y₁ wie gemäß einem beliebigen der Ansprüche 1 bis 11 definiert sind und Y₁ an den 5'-exozyklischen Kohlenstoff der Morpholino-Untereinheit angrenzt, umfasst,
wobei die Verknüpfungen den Morpholinostickstoff einer Untereinheit an den 5'-exo-zyklischen Kohlenstoff einer angrenzenden Untereinheit binden.

14. Verfahren zum Nachweisen des Vorliegens eines Polynukleotids mit einer gewählten Zielsequenz in einer Probe, umfassend das Kontaktieren eines Polymeren eines beliebigen der Ansprüche 1 bis 12 mit dem Polynukleotid, wobei das Polymer (i) eine Reihe von Basen-paarenden Resten aufweist, die zur Bindung an die gewählte Zielsequenz in der Lage sind, und (ii) mit einer nachweisbaren Reportergruppe markiert sind, und wobei das Umsetzen des Polymeren mit dem Polynukleotid unter Bedingungen durchgeführt wird, die wirksam sind, um die Bildung eines Hybridisierungskomplexes zwischen dem Polymer und der Zielsequenz zu ermöglichen, und
Nachweisen der Anwesenheit der Reportergruppe.

15. Verfahren gemäß Anspruch 14, bei dem das Polynukleotid einzelsträngig ist.

16. Verfahren gemäß Anspruch 15, bei dem das Polynukleotid DNA ist.

17. Verfahren gemäß Anspruch 15, bei dem das Polynukleotid RNA ist.

18. Verfahren gemäß Anspruch 14, bei dem das Polynukleotid doppelsträngig ist.

19. Verfahren gemäß Anspruch 14, bei dem das Polynukleotid an einen festen Träger gebunden ist.

20. Verfahren zur Isolierung einer Nukleinsäure mit einer gewählten Zielsequenz aus einer Probe, umfassend
das Kontaktieren eines Polymeren von einem beliebigen der Ansprüche 1-11 mit der Nukleinsäure, wobei das Polymer (i) eine Reihe von Basen-paarenden Resten besitzt, die zur Bindung der gewählten Zielsequenz in der Lage sind, und (ii) an einen festen Träger gebunden ist, und (iii), wobei das Kontaktieren des Polymeren mit der Nukleinsäure unter Bedingungen durchgeführt wird, die wirksam sind, um die Bildung eines Hybridisierungskomplexes zwischen dem Polymer und der Zielsequenz zu ermöglichen, und
Isolieren der Nukleinsäure.

## Revendications (Revendications pour l'(les) Etat(s) contractant(s) suivant(s): AT, BE, CH & LI, DE, DK, FR, GB, IT, LU, NL & SE)

1. Polymère constitué de structures de sous-unité morpholino de la forme : 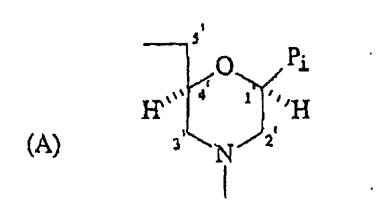 où (i) les structures sont liées ensemble par des liaisons chirales non chargées joignant l'atome d'azote du groupe morpholino d'une sous-unité à l'atome de carbone 5'-exocyclique d'une sous-unité adjacente et (ii) Pᵢ représente une partie d'appariement de base purine ou pyrimidine efficace pour se lier par liaison hydrogène spécifique de base à une base dans un polynucléotide, et où la liaison (i) est 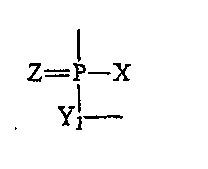 dans laquelle X représente F, CH₂R, OCH₂R, -S-CH₂R, NR₁R₂, un groupe morpholino, pyrrolyle ou pyrazolyle ; où R, R₁ et R₂ peuvent être identiques ou différents et sont choisis parmi H et CH₃ ; Y₁ est joint à l'atome de carbone 5'-exocyclique de la sous-unité morpholino ; Y₁ représente O, S, CH₂ ou NR ; et Z représente O ou S.

2. Polymère de la revendication 1, dans lequel Pᵢ est choisi parmi : 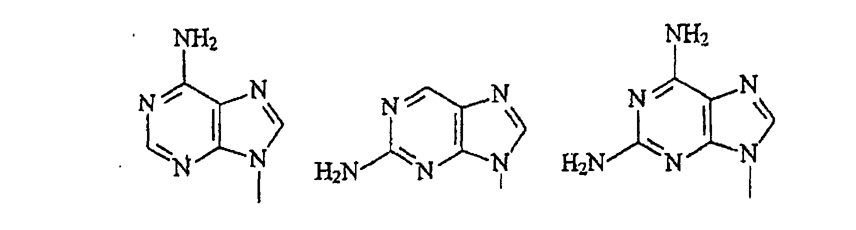 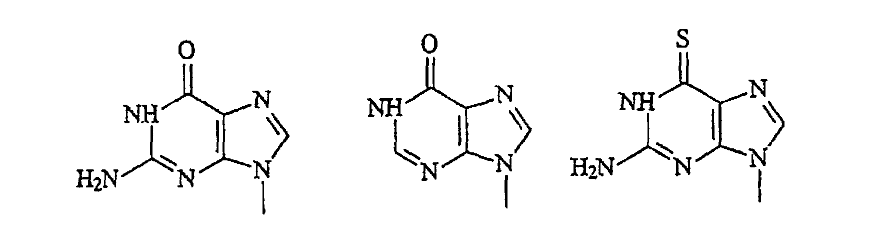 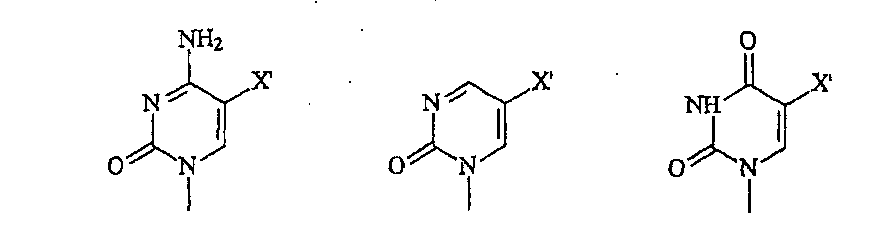 où X' représente H, CH₃, F, Cl, Br ou I.

3. Polymère de la revendication 1 ou 2, dans lequel Z représente O et Y₁ représente O.

4. Polymère de la revendication 1 ou 2, dans lequel la liaison est de la forme : 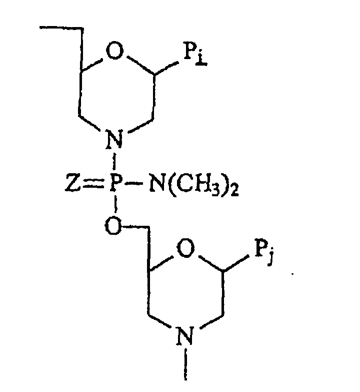 où Pⱼ représente une partie d'appariement de base purine ou pyrimidine efficace pour se lier par liaison hydrogène spécifique de base à une base dans un polynucléotide.

5. Polymère de la revendication 1 ou 2, dans lequel la liaison est de la forme : 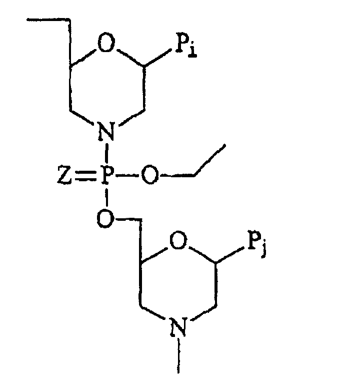 dans laquelle Pⱼ représente une partie d'appariement de base purine ou pyrimidine efficace pour se lier par liaison hydrogène spécifique de base à une base dans un polynucléotide.

6. Polymère de l'une quelconque des revendications précédentes, qui inclut en outre une partie à une ou aux deux extrémités, qui est efficace pour augmenter la solubilité dudit polymère en milieu aqueux.

7. Polymère de la revendication 6, dans lequel la partie à une ou aux deux extrémités est un polyéthylèneglycol.

8. Polymère de l'une quelconque des revendications précédentes, composé d'au moins trois sous-unités morpholino.

9. Polymère de l'une quelconque des revendications précédentes, dans lequel au moins une des parties Pᵢ est une partie 2,6-diaminopurine.

10. Polymère de l'une quelconque des revendications précédentes, dans lequel au moins une des parties Pᵢ est une partie 5-halogéno-uracile.

11. Polymère de l'une quelconque des revendications précédentes, dans lequel au moins 70% des parties Pᵢ sont des parties purine contenant un groupe 2-amine.

12. Polymère de l'une quelconque des revendications précédentes attaché à un support solide.

13. Méthode de détection, dans un échantillon, de la présence d'un polynucléotide ayant une séquence cible choisie, comprenant le fait de mettre un polymère de l'une quelconque des revendications 1 à 12 en contact avec ledit polynucléotide, où ledit polymère (i) a une série de parties d'appariement de base capables de se lier à la séquence cible choisie, et (ii) est marqué par un groupe reporter détectable, et où ladite réaction du polymère avec le polynucléotide est mise en oeuvre dans des conditions efficaces pour permettre la formation d'un complexe d'hybridation entre le polymère et la séquence cible, et de détecter la présence du groupe reporter.

14. Méthode de la revendication 13, dans laquelle ledit polynucléotide est monocaténaire.

15. Méthode de la revendication 14, dans laquelle ledit polynucléotide est un ADN.

16. Méthode de la revendication 14, dans laquelle ledit polynucléotide est un ARN.

17. Méthode de la revendication 13, dans laquelle ledit polynucléotide est bicaténaire.

18. Méthode de la revendication 13, dans laquelle ledit polynucléotide est lié à un support solide.

19. Méthode d'isolement, à partir d'un échantillon, d'un acide nucléique ayant une séquence cible choisie, comprenant le fait
de mettre un polymère de l'une quelconque des revendications 1-11 en contact avec ledit acide nucléique, ledit polymère (i) ayant une série de parties d'appariement de base capables de se lier à la séquence cible choisie et (ii) étant attaché à un support solide, et (iii) où ledit fait de mettre le polymère en contact avec l'acide nucléique est effectué dans des conditions efficaces pour permettre la formation d'un complexe d'hybridation entre le polymère et la séquence cible, et
d'isoler ledit acide nucléique.

## Revendications (Revendications pour l'(les) Etat(s) contractant(s) suivant(s): ES & GR)

1. Polymère constitué de structures de sous-unité morpholino de la forme : 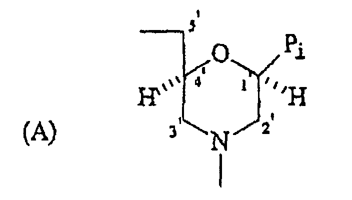 où (i) les structures sont liées ensemble par des liaisons chirales non chargées joignant l'atome d'azote du groupe morpholino d'une sous-unité à l'atome de carbone 5'-exocyclique d'une sous-unité adjacente et (ii) Pᵢ représente une partie d'appariement de base purine ou pyrimidine efficace pour se lier par liaison hydrogène spécifique de base à une base dans un polynucléotide, et où la liaison (i) est 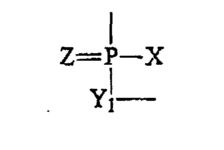 dans laquelle X représente F, CH₂R, OCH₂R, -S-CH₂R, NR₁R₂, un groupe morpholino, pyrrolyle ou pyrazolyle ; où R, R₁ et R₂ peuvent être identiques ou différents et sont choisis parmi H et CH₃ ; Y₁ est joint à l'atome de carbone 5'-exocyclique de la sous-unité morpholino ; Y₁ représente O, S, CH₂ ou NR ; et Z représente O ou S.

2. Polymère de la revendication 1, dans lequel Pᵢ est choisi parmi : 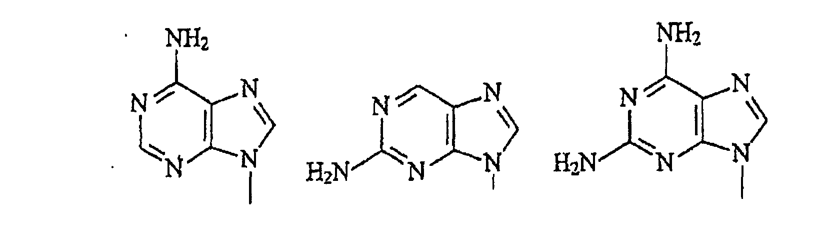 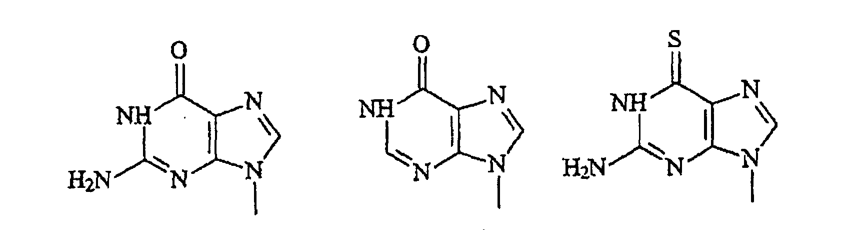 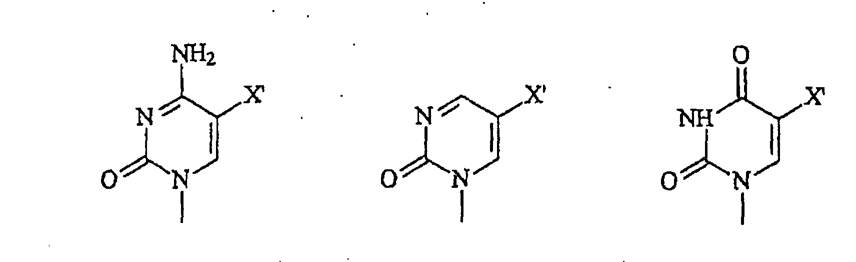 où X' représente H, CH₃, F, Cl, Br ou I.

3. Polymère de la revendication 1 ou 2, dans lequel Z représente O et Y₁ représente O.

4. Polymère de la revendication 1 ou 2, dans lequel la liaison est de la forme : 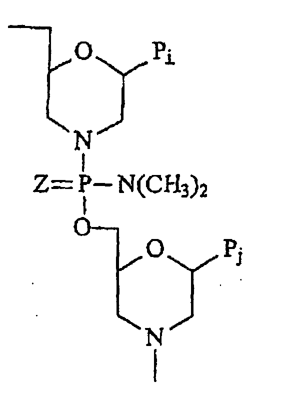 où Pⱼ représente une partie d'appariement de base purine ou pyrimidine efficace pour se lier par liaison hydrogène spécifique de base à une base dans un polynucléotide.

5. Polymère de la revendication 1 ou 2, dans lequel la liaison est de la forme : 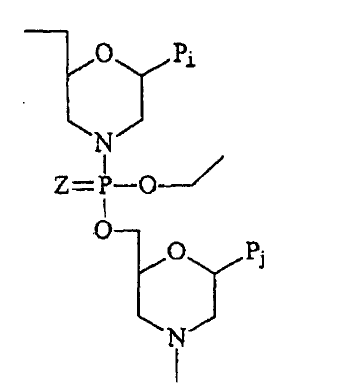 dans laquelle Pⱼ représente une partie d'appariement de base purine ou pyrimidine efficace pour se lier par liaison hydrogène spécifique de base à une base dans un polynucléotide.

6. Polymère de l'une quelconque des revendications précédentes, qui inclut en outre une partie à une ou aux deux extrémités qui est efficace pour augmenter la solubilité dudit polymère en milieu aqueux.

7. Polymère de la revendication 6, dans lequel la partie à une ou aux deux extrémités est un polyéthylèneglycol.

8. Polymère de l'une quelconque des revendications précédentes, composé d'au moins trois sous-unités morpholino.

9. Polymère de l'une quelconque des revendications précédentes, dans lequel au moins une des parties Pᵢ est une partie 2,6-diaminopurine.

10. Polymère de l'une quelconque des revendications précédentes, dans lequel au moins une des parties Pᵢ est une partie 5-halogéno-uracile.

11. Polymère de l'une quelconque des revendications précédentes, dans lequel au moins 70% des parties Pᵢ sont des parties purine contenant un groupe 2-amine.

12. Polymère de l'une quelconque des revendications précédentes attaché à un support solide.

13. Procédé de préparation d'un polymère selon l'une quelconque des revendications 1 à 11, ce procédé comprenant le fait de lier ensemble des structures de sous-unités morpholino de forme : 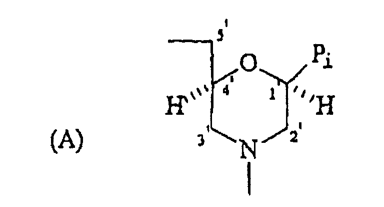 où Pᵢ est tel que défini dans l'une quelconque des revendications 1 à 11, par l'intermédiaire de liaisons chirales non chargées de formule 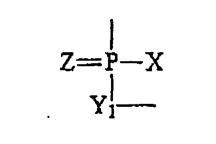 dans laquelle Z, X et Y₁ sont tels que définis dans l'une quelconque des revendications 1 à 11 et Y₁ est joint à l'atome de carbone 5'-exocyclique de la sous-unité morpholino,
ces liaisons joignant l'atome d'azote du groupe morpholino d'une sous-unité à l'atome de carbone 5'-exocyclique d'une sous-unité adjacente.

14. Méthode de détection, dans un échantillon, de la présence d'un polynucléotide ayant une séquence cible choisie, comprenant le fait de mettre un polymère de l'une quelconque des revendications 1 à 12 en contact avec ledit polynucléotide, où ledit polymère (i) a une série de parties d'appariement de base capables de se lier à la séquence cible choisie, et (ii) est marqué par un groupe reporter détectable, et où ladite réaction du polymère avec le polynucléotide est mise en oeuvre dans des conditions efficaces pour permettre la formation d'un complexe d'hybridation entre le polymère et la séquence cible, et de détecter la présence du groupe reporter.

15. Méthode de la revendication 14, dans laquelle ledit polynucléotide est monocaténaire.

16. Méthode de la revendication 15, dans laquelle ledit polynucléotide est un ADN.

17. Méthode de la revendication 15, dans laquelle ledit polynucléotide est un ARN.

18. Méthode de la revendication 14, dans laquelle ledit polynucléotide est bicaténaire.

19. Méthode de la revendication 14, dans laquelle ledit polynucléotide est lié à un support solide.

20. Méthode d'isolement, à partir d'un échantillon, d'un acide nucléique ayant une séquence cible choisie, comprenant le fait
de mettre un polymère de l'une quelconque des revendications 1-11 en contact avec ledit acide nucléique, ledit polymère (i) ayant une série de parties d'appariement de base capables de se lier à la séquence cible choisie et (ii) étant attaché à un support solide, et (iii) où ledit fait de mettre le polymère en contact avec l'acide nucléique est effectué dans des conditions efficaces pour permettre la formation d'un complexe d'hybridation entre le polymère et la séquence cible, et
d'isoler ledit acide nucléique.
